# EUROPEAN PATENT SPECIFICATION

(11) **EP 3 061 468 B1**
(45) Date of publication and mention of the grant of the patent: **09.10.2019**
(21) Application number: 16162822.7
(22) Date of filing: 25.08.2010
(51) Int. Cl.: A61L 27/38, C12N 5/071, A61L 27/54, A61L 2/02, C12N 7/00, A61L 27/36, A61L 31/00, A61L 15/40, A61K 35/44

(54) **UMBILICAL CORD AMNIOTIC MEMBRANE PRODUCTS**
AMNIOTISCHE MEMBRANPRODUKTE AUS NABELSCHNÜREN
PRODUITS DE MEMBRANE AMNIOTIQUE DE CORDON OMBILICAL

(30) Priority: 25.08.2009 US 236779 P
(43) Date of publication of application: 31.08.2016
(62) Divisional of application: 10815849.4
(73) Proprietor: Tissue Tech, Inc., Miami, FL 33173 (US)
(72) Inventor: TSENG, Scheffer, Pinecrest, Florida (US); TAN, Ek, Kia, Miami, Florida 33176 (US); TSENG, Amy, Pinecrest, Florida 33156 (US)
(74) Representative: HGF Limited

(56) References cited:
- US-A1- 2004 057 938
- US-A1- 2008 069 895
- US-B1- 6 326 019

## Description

### BACKGROUND OF THE INVENTION

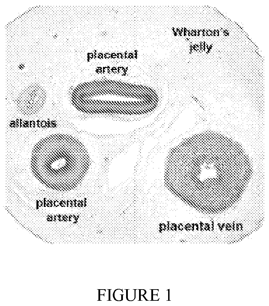

In placental mammals, the umbilical cord (i.e., the *funiculus umbilicalis*) connects the developing fetus to the placenta. The umbilical cord is made up of amniotic membrane (UCAM) and Wharton's Jelly. The UCAM functions to regulate the fluid pressure within the UC. Wharton's Jelly is a gelatinous substance within the umbilical cord, largely made up of mucopolysaccharides (hyaluronic acid and chondroitin sulfate). It also contains some fibroblasts and macrophages. The umbilical cord further comprises two arteries (the umbilical arteries) and one vein (the umbilical vein), buried within the Wharton's Jelly. For a cross-sectional view of an umbilical cord, see FIGURE 1.

US2008/069895A1 discloses biomaterials derived form umbilical cord (e.g. umbilical cord membrane = UCAM) for the repair of organs and tissues. The biomaterial comprises an isolated mammalian umbilical cord membrane. It may comprise at least one umbilical vessel or Wharton's jelly. The biomaterial comprises less water than native umbilical cord membrane in vivo, e.g. less than 40%. In other embodiments, the biomaterial comprises at least 60% water by weight. The umbilical cord membrane of the biomaterial is cut or slit longitudinally. It can be substantially flat or tubular. The biomaterial can be freeze dried.

### SUMMARY OF THE INVENTION

There is a need for a product derived from a natural source (e.g., human, non-human primate, pig or cow) that repairs, reconstructs, replaces or supplements tissue (e.g., tendons or nerves) that has been damaged, compromised, or is even missing. In order to fulfill the aforementioned needs such a product should be regenerative, anti-inflammatory, anti-scarring, anti-angiogenic, anti-adhesion, promote wound healing, durable, strong, flexible, and conformable. Such a product should also preferably serve as a niche for the *in vivo* growth and differentiation of stem cells. Although AM isolated from human placenta possesses the required biological activity (i.e., regenerative, anti-inflammatory, anti-scarring, anti-angiogenic, and anti-adhesion), placental-derived AM is thin and diaphanous. Several products are durable and strong, but do not have the required biological activity (i.e., regenerative, anti-inflammatory, anti-scarring, anti-angiogenic, and anti-adhesion).

Disclosed herein, in certain embodiments, is a UCAM product. UCAM products are regenerative, anti-inflammatory, anti-scarring, anti-angiogenic, anti-adhesion, durable, strong, flexible, and conformable. In some embodiments, the UCAM product is used as a wound covering. In some embodiments, the UCAM product is used as a patch over a device. In some embodiments, the UCAM product is used as an anti-adhesion barrier. In some embodiments, the UCAM product is used for wound repair (e.g. tendon or nerve wrapping). In some embodiments, the UCAM product is a substantially-flat sheet. In some embodiments, the UCAM product is a tubular sheet. In some embodiments, the UCAM product is pulverized.

Disclosed herein, in some embodiments, is a composition, comprising: an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum,* wherein the natural structural integrity of the isolated UCAM is substantially preserved for at least 15 days after initial procurement. In some embodiments, the composition has higher yield strength, higher stiffness, higher pull strength, higher tensile strength, and higher suture pull-out strength than a composition comprising placental amniotic membrane (PAM). In some embodiments, the natural biological activity of the isolated UCAM is substantially preserved for at least 15 days after initial procurement. In some embodiments, the composition is anti-inflammatory, anti-scarring, anti-angiogenic, anti-adhesion, or promotes wound healing when contacted with an exogenous living cell. In some embodiments, substantially all red blood cells have been removed from the UCAM. In some embodiments, the composition is cryopreserved, lyophilized, terminally sterilized, or a combination thereof. In some embodiments, the composition is substantially-flattened sheet. In some embodiments, the composition is a tubular sheet. In some embodiments, the composition is a pulverized powder or a homogenate.

Disclosed herein, in some embodiments, is a method of producing a UCAM product, comprising: obtaining pre-frozen umbilical cord, and separating the UCAM from the umbilical vein and umbilical arteries and at least a portion of the Wharton's Jelly, wherein the natural structural integrity of the UCAM product is substantially preserved for at least 15 days after initial procurement. In some embodiments, the natural biological activity of the isolated UCAM is substantially preserved for at least 15 days after initial procurement. In some embodiments, the umbilical cord is obtained from a human, non-primate human, cow or pig. In some embodiments, the UCAM product is anti-inflammatory, anti-scarring, anti-angiogenic, anti-adhesion, or promotes wound healing when contacted with an exogenous living cell. In some embodiments, the UCAM product has higher yield strength, higher stiffness, higher pull strength, higher tensile strength, and higher suture pull-out strength than a composition comprising placental amniotic membrane (PAM). In some embodiments, the UCAM is separated from the umbilical vein and umbilical arteries and at least a portion of the Wharton's Jelly by use of a surgical dermatome. In some embodiments, the method further comprises inhibiting the metabolic activity of substantially all cells found on the UCAM by freezing or drying the umbilical cord. In some embodiments, the method further comprises draining blood from the umbilical cord before removing Wharton's Jelly, the umbilical vein, and the umbilical arteries. In some embodiments, the method further comprises removing substantially all red blood cells from the UCAM. In some embodiments, the method further comprises lyophilizing, cryopreserving, pulverizing, or terminally sterilizing the UCAM product.

Disclosed herein, in certain embodiments, is the use of a UCAM product disclosed herein to promote wound healing and reduce inflammation, scarring, angiogenesis, and adhesion in a plurality of exogenous cells, wherein the exogenous cells are contacted with a UCAM product comprising an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum.*

Disclosed herein, in certain embodiments, is the use of a UCAM product disclosed herein as a wound covering for an injured tissue, wherein the injured tissue is contacted with a UCAM product comprising an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum.*

Disclosed herein, in certain embodiments, is the use of a UCAM product disclosed herein for repairing or supplementing damaged tissue, wherein the damaged tissue is contacted with a UCAM product comprising an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum.*

Disclosed herein, in certain embodiments, is the use of a UCAM product disclosed herein as an anti-adhesion barrier, wherein the tissue to be protected from adhesion is contacted with a UCAM product comprising an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum.*

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIGURE 1 is a cross-section of an umbilical cord (UC).

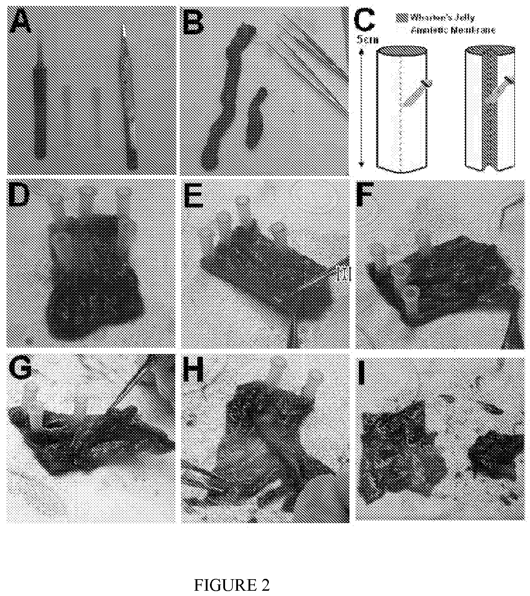

FIGURE 2 illustrates the protocol for generating tissue grafts from UCAM.
FIGURE 3a demonstrates a method for flattening the umbilical cord. The umbilical cord is cut longitudinally (dotted lines) using a scalpel without cutting it into two halves. Additional cuts are made (dotted lines) into the Wharton's Jelly to flatten out the tubular UC. FIGURE 3b demonstrates a method of securing flattened umbilical cord to a substrate.
FIGURE 4 illustrates Day 0, t=3h (A-C: UCAM in Preservation Solution (50% DMEM + 50% Glycerol), D-F: UCAM spread out on dish).

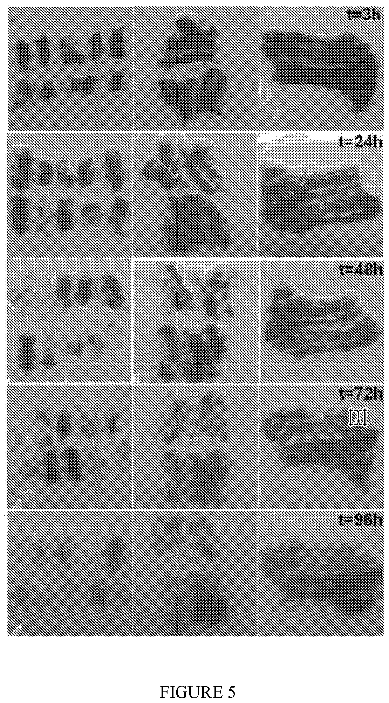

FIGURE 5 illustrates UCAM after being kept in preservation media for t=3h, 24h, 48h, 72h and 96h. The image has been gray scaled. The grayer the tissue sample, the more blood there is in the tissue sample. For instance, at three hours the tissue samples are dark grey as none of the red blood cells have diffused from the tissue. However, 96 hours of exposure to preservation media results in diffusion of substantially all of the red blood cells from the tissue and nearly clear tissue samples.

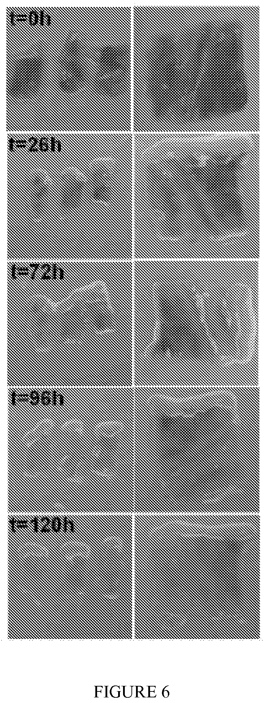

FIGURE 6 illustrates UCAM after 5 days in preservation media. The image has been gray scaled. The grayer the tissue sample, the more blood there is in the tissue sample. For instance, at zero hours the tissue samples are dark grey as none of the red blood cells have diffused from the tissue. However, 120 hours of exposure to preservation media results in diffusion of substantially all of the red blood cells from the tissue and nearly clear tissue samples.

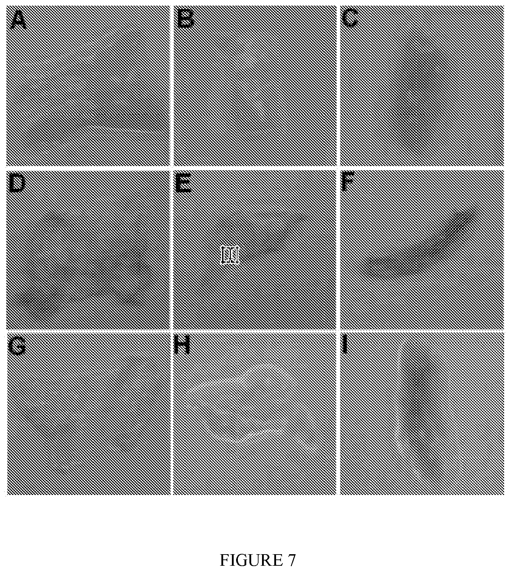

FIGURE 7 illustrates UCAM after 24h in PBS 1X (A-C), UCAM after lyophilization (D-F) and UCAM after rehydration in 50% DMEM+50% Glycerol (G-I).
FIGURE 8 illustrates UCAM after lyophilization and rehydration.
FIGURE 9 compares UCAM processed from umbilical cord drained of blood (bUC) (CB09989) and umbilical cord not drained of blood (UC) (UB085017). The image has been gray scaled. The amount of blood in the tissue graft is indicated by the amount of gray in the tissue. For example, the processed bUC has a lighter color than the processed UC - this indicates that bUC results in a clearer product than UC.
FIGURE 10 compares the efficiency of removing blood stain with PBS 1X and 50% DMEM + 50% Glycerol. The image has been gray scaled. The grayer the tissue sample, the more blood there is in the tissue sample. For instance, at zero hours the tissue samples are darker grey than the tissue samples after 16 hours of diffusion.

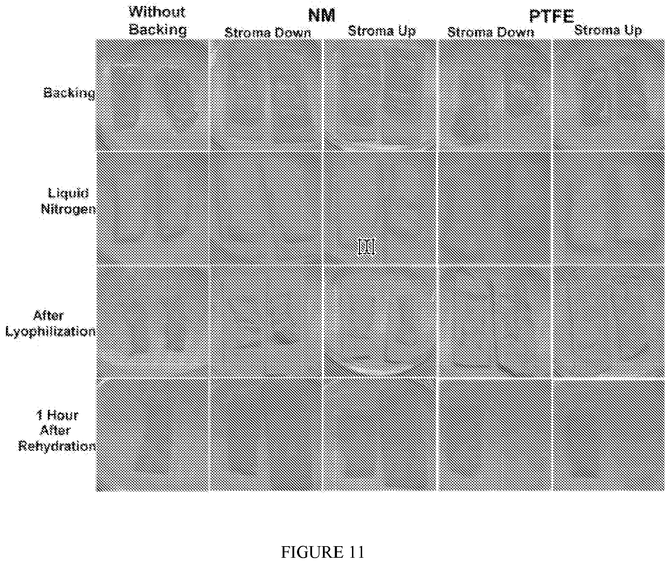

FIGURE 11 illustrates UCAM after freezing, lyophilization and rehydration with PBS 1X.
FIGURE 12 illustrates the protein concentration in five extracts made from biological material containing UCAM (AM-UCAM; CH-chorionic membrane; AC-both AM and CH; UC-umbilical cord; PL-placenta).
FIGURE 13 illustrates the cell viability of RAW264.7 cells when treated simultaneously (A and B) or sequentially (C and D) with 200 µg/ml protein in each extract without or with 1 µg/ml LPS stimulation.
FIGURE 14A presents the results of a Western blot analysis for HC1 and HC3 for AM extract (Ext. A). FIGURE 14B presents the results of a Western Blot analysis for bikunin of |α| in AME, CHE, Placental Extract, and Umbilical Cord Extract. FIGURE 14C presents the results of a Western Blot analysis for PTX3 in AME, CHE, Placental Extract, and Umbilical Cord Extract.

### DETAILED DESCRIPTION OF THE INVENTION

There is a need for a product derived from a natural source (e.g., human, non-human primate, pig or cow) that repairs, reconstructs, replaces or supplements tissue (e.g., tendons or nerves) that has been damaged, compromised, or is even missing. In order to fulfill the aforementioned needs such a product should be regenerative, anti-inflammatory, anti-scarring, anti-angiogenic, anti-adhesion, promote wound healing, durable, strong, flexible, and conformable. Such a product should also preferably serve as a niche for the *in vivo* growth and differentiation of stem cells. Although AM isolated from human placenta possesses the required biological activity (i.e., regenerative, anti-inflammatory, anti-scarring, anti-angiogenic, and anti-adhesion), placental-derived AM is thin and diaphanous. Several products are durable and strong, but do not have the required biological activity (i.e., regenerative, anti-inflammatory, anti-scarring, anti-angiogenic, and anti-adhesion).

Disclosed herein, in certain embodiments, is a UCAM product. UCAM products are derived from a natural source (e.g., human, non-human primate, cow or pig) regenerative, anti-inflammatory, anti-scarring, anti-angiogenic, anti-adhesion, durable, strong, flexible, and conformable. In some embodiments, the UCAM product is used as a wound covering. In some embodiments, the UCAM product is used as a patch over a device. In some embodiments, the UCAM product is used as an anti-adhesion barrier. In some embodiments, the UCAM product is used for wound repair. In some embodiments, the UCAM product is a substantially-flat sheet. In some embodiments, the UCAM product is a tubular sheet. In some embodiments, the UCAM product is pulverized.

### Certain Definitions

As used herein, "human cells, tissues, or cellular or tissue-based products (HCT/Ps)" means articles containing or consisting of human cells or tissues that are intended for implantation, transplantation, infusion, or transfer into a human recipient.

As used herein, "human tissue" means any tissue derived from a human body. In some embodiments, the human tissue is UCAM.

As used herein, "tissue graft" means a matrix of proteins (e.g., collagen and elastin) and glycans (e.g., dermatan, hyaluronan, and chondroitin) that is used to replace damaged, compromised, or missing tissue. In certain instances, the matrix is laid down and host cells gradually integrate into the matrix.

As used herein, "minimal manipulation" means (1) for structural tissue, processing that does not alter the original relevant characteristics of the tissue relating to the tissue's utility for reconstruction, repair, or replacement; and (2) for cells or nonstructural tissues, processing that does not alter the relevant biological characteristics of cells or tissues.

As used herein, 'homologous use" means the repair, reconstruction, replacement, or supplementation of a recipient's cells or tissues with an HCT/P that performs the same basic function or functions in the recipient as in the donor.

As used herein, "processing" means any activity performed on an HCT/P, other than recovery, donor screening, donor testing, storage, labeling, packaging, or distribution, such as testing for microorganisms, preparation, sterilization, steps to inactivate or remove adventitious agents, preservation for storage, and removal from storage.

As used herein, a "culture," refers to the cultivation or growth of cells, for example, tissue cells, in or on a nutrient medium. As is well known to those of skill in the art of cell or tissue culture, a cell culture is generally begun by removing cells or tissue from a human or other animal, dissociating the cells by treating them with an enzyme, and spreading a suspension of the resulting cells out on a flat surface, such as the bottom of a Petri dish. There the cells generally form a thin layer of cells called a "mono-layer" by producing glycoprotein-like material that causes the cells to adhere to the plastic or glass of the Petri dish. A layer of culture medium, containing nutrients suitable for cell growth, is then placed on top of the mono-layer, and the culture is incubated to promote the growth of the cells.

As used herein, "sheet" means any continuous expanse or surface. In some embodiments, a sheet of UCAM is flat. In some embodiments, a sheet of UCAM is tubular. The sheet can be any shape. In some embodiments, the sheet is a square, circle, triangle, or rectangle.

As used herein, the term "subject" is used to mean any animal, preferably a mammal, including a human or non-human. The terms patient, subject, and individual are used interchangeably. None of the terms are to be interpreted as requiring the supervision of a medical professional (e.g., a doctor, nurse, physician's assistant, orderly, hospice worker).

"Substantially isolated" or "isolated" when used in the context of UCAM means that the UCAM is separated from most other non-UCAM materials (e.g., red blood cells, blood vessels, and arteries) derived from the original source organism.

The terms "treat," "treating" or "treatment," as used herein, include alleviating, abating or ameliorating a disease or condition symptoms, preventing additional symptoms, ameliorating or preventing the underlying metabolic causes of symptoms, inhibiting the disease or condition, e.g., arresting the development of the disease or condition, relieving the disease or condition, causing regression of the disease or condition, relieving a condition caused by the disease or condition, or stopping the symptoms of the disease or condition either prophylactically and/or therapeutically.

As used herein, the terms "durable" and "strong" mean that UCAM is an elastic material with higher yield strength, higher stiffness, higher pull strength, higher tensile strength, and higher suture pull-out strength than placental amniotic membrane (PAM).

As used herein, the phrase "wherein the biological and structural integrity of the isolated UCAM is substantially preserved" means that when compared to the biological activity and structural integrity of fresh UCAM, the biological activity and structural integrity of the isolated UCAM has only decreased by about 5%, about 10%, about 15%, about 20%, about 25%, about 30%, about 35%, about 40%, about 50%, about 60%.

The term "fresh UCAM" refers to UCAM that is less than 10 days old following birth, and which is in substantially the same form as it was following birth.

As used herein, "biological activity" means the activity of polypeptides and polysaccharides. In some embodiments, the activity of polypeptides and polysaccharides found in UCAM (and isolated UCAM) is anti-inflammatory, anti-scarring, anti-angiogenic, or anti-adhesion. In some embodiments, the activity of polypeptides and polysaccharides found in UCAM (and isolated UCAM) promotion of wound healing.

As used herein, "structural integrity" means the integrity of stroma and basement membrane that make up the UCAM. In some embodiments, the structural integrity of the UCAM results in suture pull out strength.

### The Umbilical Cord

In placental mammals, the umbilical cord (the UC; i.e., the *funiculus umbilicalis*) connects the developing fetus to the placenta. The umbilical cord is made up of amniotic membrane (UCAM) and Wharton's Jelly. The UCAM functions to regulate the fluid pressure within the UC. For a cross-sectional view of an umbilical cord, see FIGURE 1.

As used herein, "Wharton's Jelly" means a gelatinous substance within the umbilical cord, largely made up of mucopolysaccharides (hyaluronic acid and chondroitin sulfate).

The umbilical cord further comprises two arteries (the umbilical arteries) and one vein (the umbilical vein), buried within the Wharton's jelly. In certain instances, an umbilical vein supplies a developing fetus with oxygenated blood from the placenta. In certain instances, an umbilical artery returns deoxygenated blood to the placenta.

### Umbilical Cord UCAM (UCAM)

UCAM is a translucent membrane. The UCAM has multiple layers-an epithelial layer, a basement membrane; a compact layer; a fibroblast layer; and a spongy layer. It lacks blood vessels or a direct blood supply. UCAM lacks the immunogenic antigens HLA-A, B, or DR - reducing the risk of transplant rejection. Further, UCAM is rich in IL-6, IL-8, EGF, TGF-α, KGF, HGF, bFGF, TGF-β1, TGF- β 2, endothelin-1, leukotrienes, CA-1, and CA-2.

UCAM possesses several regenerative properties. It reduces inflammation, reduces angiogenesis, reduces scarring, and reduces adhesion. Further, the basement membrane of the UCAM serves as a natural niche for stem cells. Thus, wounds covered with a UCAM products often display an increased rate of healing as compared to wounds covered with a tissue graft made of alternative materials.

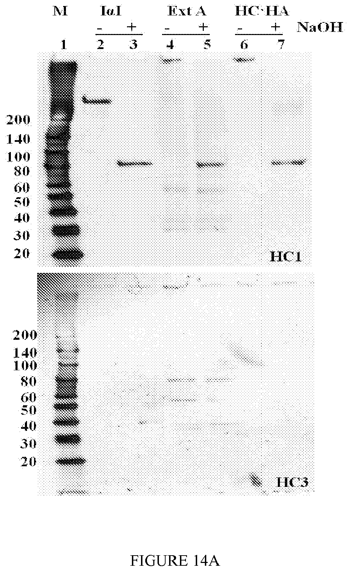

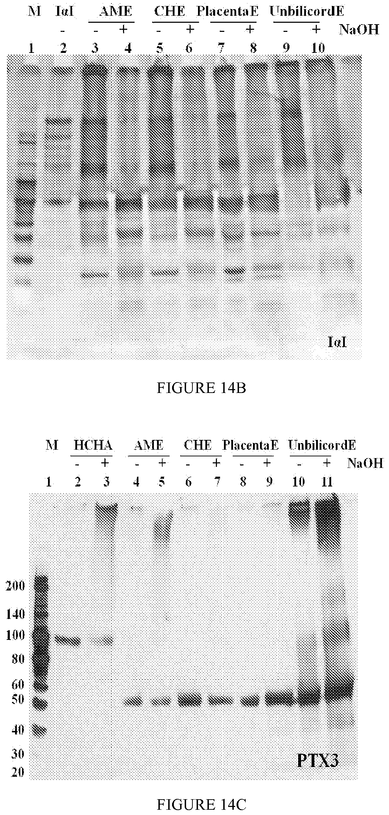

As shown in FIGURE 14, UCAM contains |α| (Inter-α-trypsin inhibitor) and PTX-3. |α| is a composite protein containing a light chain (called urinary trypsin inhibitor [UTI] or bikunin), and two heavy chains that can bind HA. Figure 14C also shows that UCAM contains a greater concentration of PTX-3 than any other extract containing UCAM. lαl and PTX-3 contribute to the anti-inflammatory, anti-scarring, anti-angiogenic, and anti-adhesion properties of UCAM.

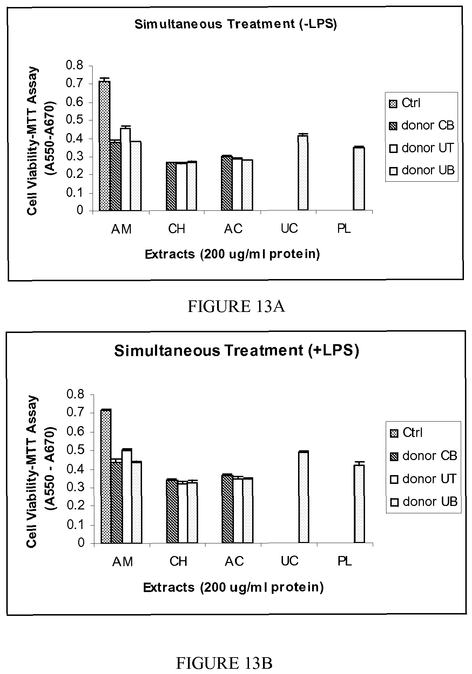

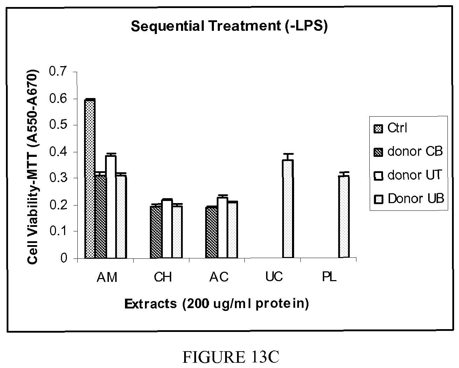

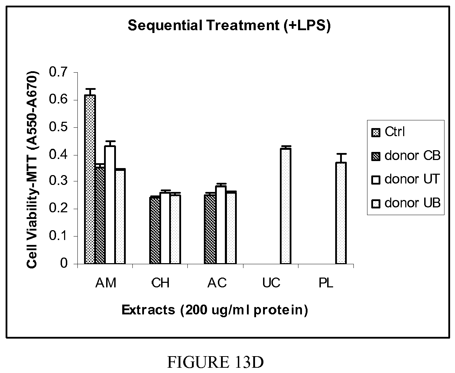

As shown in FIGURE 13, UCAM reduces the viability of macrophages. In some embodiments, UCAM reduces the viability of macrophages by reducing actin filaments, decreasing the secretion of TNF-a and IL-6, increasing the secretion of IL-10, suppressing activation of macrophages, and inducing apoptosis in macrophages. In some embodiments, reducing the viability of macrophages reduces scarring and inflammation.

The majority of placental AM (PAM) is highly elastic and thin (e.g., about 75 microns thick). PAM found adjacent to the umbilical cord is durable and strong - unfortunately, this area is extremely small. UCAM is stronger, thicker (about 300 to about 1500 microns thick), more flexible, more conformable, and less stretchable than AM found elsewhere (e.g., PAM) - making it more durable and thus useful for supplementing tissue (e.g., epidermis and soft tissue).

Problematically, UCAM is highly inaccessible due to its strong association with Wharton's Jelly and the curvature of the umbilical cord. Further, UCAM contains significant amounts of red blood cells that result in cosmetically undesirable tissue grafts. The inventors have solved the aforementioned problems associated with UCAM by formulating precise protocols that enable useful tissue grafts to be made from this previously unusable material.

### Generation of Flat UCAM Sheets

Disclosed herein, in some embodiments, is a flat UCAM sheet, comprising: an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum,* wherein the natural structural integrity of the isolated UCAM is substantially preserved for at least 15 days after initial procurement. In some embodiments, the biological and structural integrity of the isolated UCAM is substantially preserved for at least 20 days after initial procurement. In some embodiments, the biological and structural integrity of the isolated UCAM is substantially preserved for at least 25 days after initial procurement. In some embodiments, the biological and structural integrity of the isolated UCAM is substantially preserved for at least 30 days after initial procurement. In some embodiments, the biological and structural integrity of the isolated UCAM is substantially preserved for at least 35 days after initial procurement. In some embodiments, the biological and structural integrity of the isolated UCAM is substantially preserved for at least 40 days after initial procurement. In some embodiments, the biological and structural integrity of the isolated UCAM is substantially preserved for at least 45 days after initial procurement. In some embodiments, the biological and structural integrity of the isolated UCAM is substantially preserved for at least 50 days after initial procurement. In some embodiments, the biological and structural integrity of the isolated UCAM is substantially preserved for at least 55 days after initial procurement. In some embodiments, the biological and structural integrity of the isolated UCAM is substantially preserved for at least 60 days after initial procurement.

Further disclosed herein, in certain embodiments, a method of producing a flat UCAM sheet, comprising: obtaining pre-frozen umbilical cord, and separating the UCAM from the umbilical vein and umbilical arteries and at least a portion of the Wharton's Jelly, wherein the structural integrity of the UCAM product is substantially preserved for at least 15 days after initial procurement. In some embodiments, the biological and structural integrity of the isolated UCAM is substantially preserved for at least 20 days after initial procurement. In some embodiments, the biological and structural integrity of the isolated UCAM is substantially preserved for at least 25 days after initial procurement. In some embodiments, the biological and structural integrity of the isolated UCAM is substantially preserved for at least 30 days after initial procurement. In some embodiments, the biological and structural integrity of the isolated UCAM is substantially preserved for at least 35 days after initial procurement. In some embodiments, the biological and structural integrity of the isolated UCAM is substantially preserved for at least 40 days after initial procurement. In some embodiments, the biological and structural integrity of the isolated UCAM is substantially preserved for at least 45 days after initial procurement. In some embodiments, the biological and structural integrity of the isolated UCAM is substantially preserved for at least 50 days after initial procurement. In some embodiments, the biological and structural integrity of the isolated UCAM is substantially preserved for at least 55 days after initial procurement. In some embodiments, the biological and structural integrity of the isolated UCAM is substantially preserved for at least 60 days after initial procurement.

Umbilical cord is recovered from any suitable source (e.g., a hospital or tissue bank). Umbilical cord can be obtained from any mammal, such as a human, non-human primate, cow or pig.

It is kept at -80°C until donor and specimen eligibility has been determined. In some embodiments, storing the umbilical cord at -80°C kills substantially all cells found in the umbilical cord. In some embodiments, storing the umbilical cord at -80°C kills substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM. In some embodiments, storing the umbilical cord at -80°C results in the loss of metabolic activity in substantially all cells found in the umbilical cord. In some embodiments, storing the umbilical cord at -80°C results in the loss of metabolic activity in substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM. In some embodiments, the umbilical cord is dried. In some embodiments, drying the umbilical cord kills substantially all cells found in the umbilical cord. In some embodiments, the umbilical cord is dried. In some embodiments, drying the umbilical cord results in the loss of metabolic activity in substantially all cells found in the umbilical cord.

### Initial Processing of UC

All processing is done following Good Tissue Practices (GTP) to ensure that no contaminants are introduced into the UCAM products.

The umbilical cord is tested for HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* using FDA licensed screening test. Any indication that the tissue is contaminated with HIV-1, HIV-2, HTLV-1, hepatitis B and C, West Nile virus, or cytomegalovirus results in the immediate quarantine and subsequent destruct of the tissue specimen.

Further, the donor's medical records are examined for risk factors for and clinical evidence of hepatitis B, hepatitis C, or HIV infection. Any indication that the donor has risk factors for, and/or clinical evidence of, infection with HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* results in the immediate quarantine and subsequent destruct of the tissue specimen.

The umbilical cord is frozen.

In some embodiments, substantially all of the blood is removed from the UC. In some embodiments, substantially all of the blood is removed from the umbilical cord before the umbilical cord is frozen. In instances where a tissue graft will be visible (e.g., a cosmetically desirable epidermal graft), substantially all of the blood is removed from the umbilical cord (e.g., from any arteries and veins found in the UC). In some embodiments, blood is not removed from the UC. In some embodiments, blood is not removed from the umbilical cord before the umbilical cord is frozen. In some embodiments, where a tissue graft will not be visible (e.g., the tissue graft is applied to an internal organ), the blood is not removed from the UC.

In some embodiments, the umbilical cord tissue is washed with buffer with agitation to remove excess blood and tissue. In some embodiments, washing with agitation reduces the wash time.

In some embodiments, the umbilical cord is washed with an isotonic buffer or tissue culture media. In some embodiments, the umbilical cord is washed with saline. In some embodiments, the umbilical cord is washed with PBS. In some embodiments, the umbilical cord is washed with PBS 1X. In some embodiments, the umbilical cord is washed with a TRIS-buffered saline. In some embodiments, the umbilical cord is washed with a HEPES -buffered saline. In some embodiments, the umbilical cord is washed with Ringer's solution. In some embodiments, the umbilical cord is washed with Hartmann's solution. In some embodiments, the umbilical cord is washed with EBSS. In some embodiments, the umbilical cord is washed with HBSS. In some embodiments, the umbilical cord is washed with Tyrode's Salt Solution. In some embodiments, the umbilical cord is washed with Gey's Balanced Salt Solution. In some embodiments, the umbilical cord is washed with DMEM. In some embodiments, the umbilical cord is washed with EMEM. In some embodiments, the umbilical cord is washed with GMEM. In some embodiments, the umbilical cord is washed with RPMI.

In some embodiments, the umbilical cord is cut into multiple sections (e.g., using a scalpel). The size of the sections depends on the desired use of the product (e.g., tissue graft) derived from the umbilical cord.

### Processing Method 1 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, a section of the umbilical cord is then cut longitudinally (e.g., using a scalpel or scissors). In some embodiments, the section of the umbilical cord is not cut into halves. In some embodiments, the section of the umbilical cord is cut into two halves. Optionally, in some embodiments, additional cuts are made in the Wharton's Jelly to help flatten out the UC.

In some embodiments, the cut umbilical cord tissue is optionally washed again with buffer to further remove excess blood and tissue.

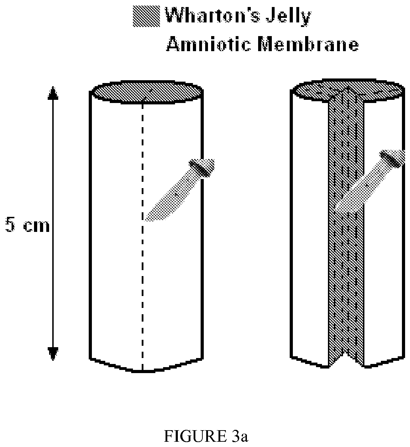

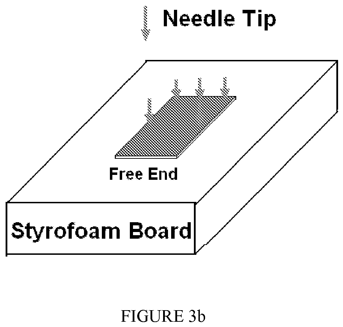

In some embodiments, the umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape) In some embodiments, the umbilical cord is orientated such that the inside face of the umbilical cord (e.g., the face comprising the Wharton's Jelly) is facing up while the outside face (i.e., the face comprising UCAM) is facing the substrate. Optionally, in some embodiments, one end of the umbilical cord is left free (see FIGURE 3b). Alternatively, in some embodiments, both ends of the umbilical cord are left free.

If the umbilical cord does not lay flat against the substrate, in some embodiments, additional cuts are made in the Wharton's Jelly.

In some embodiments, part or all of the Wharton's Jelly is removed from the UCAM. The desired thickness of the tissue graft determines how much of the Wharton's Jelly is removed. In some embodiments, the Wharton's Jelly is peeled from the umbilical cord in layers (e.g., using a set of forceps, hemostats). In some embodiments, the Wharton's Jelly is cut away (e.g., shaved) from the umbilical cord in sections. In some embodiments, a rotoblator (i.e., a catheter attached to a drill with a diamond coated burr) is utilized to remove the Wharton's Jelly. In some embodiments, a liposuction machine is utilized to remove the Wharton's Jelly. In some embodiments, a liquid under high pressure is applied to remove the Wharton's Jelly. In some embodiments, a brush is utilized to remove the Wharton's Jelly (e.g., a mechanized brush rotating under high speed). In some embodiments, UCAM is retrieved using a surgical dermatome.

In some embodiments, both ends of the umbilical cord are attached to the substrate. In some embodiments, only one end is attached to the substrate. *See* FIGURE 3b. If one end of the umbilical cord is left free, in some embodiments, the free end of the umbilical cord is held (e.g., with a clamp, hemostats or a set of forceps (e.g., wide serrated tip forceps)) while part or all of the Wharton's Jelly is removed.

The umbilical cord comprises two arteries (the umbilical arteries) and one vein (the umbilical vein). In some embodiments, the vein and arteries are removed from the umbilical cord. In certain instances, the vein and arteries are surrounded (or suspended or buried) within the Wharton's Jelly. In some embodiments, the vein and arteries are removed concurrently with the removal of the Wharton's Jelly. In some embodiments, the vein and arteries are peeled (or pulled) from the umbilical cord (e.g., using a set of forceps). In some embodiments, the vein and arteries are cut away (e.g., shaved) from the umbilical cord in sections. In some embodiments, a rotoblator removes the vein and arteries concurrently with the Wharton's Jelly. In some embodiments, a liposuction machine is utilized to remove the vein and arteries concurrently with the Wharton's Jelly. In some embodiments, a vein stripper is utilized to remove the vein and arteries concurrently with the Wharton's Jelly. In some embodiments, a liquid under high pressure removes the vein and arteries concurrently with the Wharton's Jelly. In some embodiments, a brush removes the vein and arteries concurrently with the Wharton's Jelly. In some embodiments, a surgical dermatome removes the vein and arteries concurrently with the Wharton's Jelly.

After substantially pure UCAM has been obtained, the UCAM is optionally washed with buffer to remove excess blood and tissue.

### Processing Method 2 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, whole umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape). In some embodiments, UCAM is removed directly from the tubular umbilical cord. In some embodiments, UCAM is shaved off of the umbilical cord. In some embodiments, UCAM is shaved off of the umbilical cord using any suitable method. In some embodiments, UCAM is shaved off of the umbilical cord using a shaver or a surgical dermatome.

After substantially pure UCAM has been obtained, the UCAM is optionally washed with buffer to remove excess blood and tissue.

### Processing to Generate Flat UCAM Sheets

In some embodiments, the UCAM is used to generate flat UCAM sheets. In some embodiments, the sheets are in any suitable shape (e.g., a square, a circle, a triangle, a rectangle).

The size of the UCAM sheet depends on the desired use of the UCAM sheet. In some embodiments, the UCAM is divided into sections that are about 1.0 cm x about 0.25 cm. In some embodiments, the UCAM is divided into sections that are about 1.0 cm x about 0.5 cm. In some embodiments, the UCAM is divided into sections that are about 1.0 cm x about 0.75 cm. In some embodiments, the UCAM is divided into sections that are about 1 cm x about 1 cm. In some embodiments, the UCAM is divided into sections that are about 1 cm x about 2 cm. In some embodiments, the UCAM is divided into sections that are about 1 cm x about 3 cm. In some embodiments, the UCAM is divided into sections that are about 1 cm x about 4 cm. In some embodiments, the UCAM is divided into sections that are about 1 cm x about 5 cm. In some embodiments, the UCAM is divided into sections that are about 1 cm x about 6 cm. In some embodiments, the UCAM is divided into sections that are about 2 cm x about 2 cm. In some embodiments, the UCAM is divided into sections that are about 2 cm x about 3 cm. In some embodiments, the UCAM is divided into sections that are about 2 cm x about 4 cm. In some embodiments, the UCAM is divided into sections that are about 2 cm x about 5 cm. In some embodiments, the UCAM is divided into sections that are about 2 cm x about 6 cm. In some embodiments, the UCAM is divided into sections that are about 3 cm x about 3 cm. In some embodiments, the UCAM is divided into sections that are about 3 cm x about 4 cm. In some embodiments, the UCAM is divided into sections that are about 3 cm x about 5 cm. In some embodiments, the UCAM is divided into sections that are about 3 cm x about 6 cm. In some embodiments, the UCAM is divided into sections that are about 4 cm x about 4 cm. In some embodiments, the UCAM is divided into sections that are about 4 cm x about 5 cm. In some embodiments, the UCAM is divided into sections that are about 4 cm x about 6 cm. In some embodiments, the UCAM is divided into sections that are about 5 cm x about 5 cm. In some embodiments, the UCAM is divided into sections that are about 5 cm x about 6 cm. In some embodiments, the UCAM is divided into sections that are about 6 cm x about 6 cm.

In some embodiments, the UCAM sheets are contacted with a buffer to remove substantially all remaining red blood cells. In some embodiments, the UCAM sheets are contacted with an isotonic buffer. In some embodiments, the UCAM sheets are contacted with saline. In some embodiments, the UCAM sheets are contacted with PBS. In some embodiments, the UCAM sheets are contacted with PBS 1X. In some embodiments, the UCAM sheets are contacted with Ringer's solution. In some embodiments, the UCAM sheets are contacted with Hartmann's solution. In some embodiments, the UCAM sheets are contacted with a TRIS-buffered saline. In some embodiments, the UCAM sheets are contacted with a HEPES-buffered saline. In some embodiments, the UCAM sheets are contacted with EBSS. In some embodiments, the UCAM sheets are contacted with HBSS. In some embodiments, the UCAM sheets are contacted with Tyrode's salt Solution. In some embodiments, the UCAM sheets are contacted with Gey's Balanced Salt Solution. In some embodiments, the UCAM sheets are contacted with DMEM. In some embodiments, the UCAM sheets are contacted with EMEM. In some embodiments, the UCAM sheets are contacted with GMEM. In some embodiments, the UCAM sheets are contacted with RPMI.

In some embodiments, the UCAM sheets are contacted with a buffer to remove substantially all remaining red blood cells. In some embodiments, the UCAM sheets are contacted with buffer under agitation to remove substantially all remaining red blood cells. In some embodiments, the UCAM sheets are contacted with a buffer for 10 minutes. In some embodiments, the UCAM sheets are contacted with a buffer for 15 minutes. In some embodiments, the UCAM sheets are contacted with a buffer for 20 minutes. In some embodiments, the UCAM sheets are contacted with a buffer for 25 minutes. In some embodiments, the UCAM sheets are contacted with a buffer for 30 minutes. In some embodiments, the UCAM sheets are contacted with a buffer for 35 minutes. In some embodiments, the UCAM sheets are contacted with a buffer for 40 minutes. In some embodiments, the UCAM sheets are contacted with a buffer for 45 minutes. In some embodiments, the UCAM sheets are contacted with a buffer for 50 minutes. In some embodiments, the UCAM sheets are contacted with a buffer for 55 minutes. In some embodiments, the UCAM sheets are contacted with a buffer for 60 minutes. In some embodiments, the UCAM sheets are contacted with a buffer for 2 hours. In some embodiments, the UCAM sheets are contacted with a buffer for 3 hours. In some embodiments, the UCAM sheets are contacted with a buffer for 4 hours. In some embodiments, the UCAM sheets are contacted with a buffer for 5 hours. In some embodiments, the UCAM sheets are contacted with a buffer for 6 hours. In some embodiments, the UCAM sheets are contacted with a buffer for 6 hours. In some embodiments, the UCAM sheets are contacted with a buffer for 10 hours. In some embodiments, the UCAM sheets are contacted with a buffer for 12 hours. In some embodiments, the UCAM sheets are contacted with a buffer for 18 hours. In some embodiments, the UCAM sheets are contacted with a buffer for 24 hours. In some embodiments, the UCAM sheets are contacted with a buffer for 2 days. In some embodiments, the UCAM sheets are contacted with a buffer for 3 days. In some embodiments, the UCAM sheets are contacted with a buffer for 4 days. In some embodiments, the UCAM sheets are contacted with a buffer for 5 days. In some embodiments, the UCAM sheets are contacted with a buffer for 6 days. In some embodiments, the UCAM sheets are contacted with a buffer for 7 days. In some embodiments, the buffer is optionally changed during the contacting (e.g., when the rate at which red blood cells diffuse from the UCAM sheets slows). In some embodiments, a magnetic stirrer is added during the contacting. In some embodiments, adding (and activating) a magnetic stirrer increases the rate at which the red blood cells diffuse from the UCAM sheets.

### Generation of Tubular UCAM Sheets

Disclosed herein, in some embodiments, is a tubular UCAM sheet, comprising: an isolated UCAM that does not comprise a vein or an artery, a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum,* wherein the natural structural integrity of the isolated UCAM is substantially preserved for at least 15 days after initial procurement. In some embodiments, the biological and structural integrity of the isolated UCAM is substantially preserved for at least 20 days after initial procurement. In some embodiments, the biological and structural integrity of the isolated UCAM is substantially preserved for at least 25 days after initial procurement. In some embodiments, the biological and structural integrity of the isolated UCAM is substantially preserved for at least 30 days after initial procurement. In some embodiments, the biological and structural integrity of the isolated UCAM is substantially preserved for at least 35 days after initial procurement. In some embodiments, the biological and structural integrity of the isolated UCAM is substantially preserved for at least 40 days after initial procurement. In some embodiments, the biological and structural integrity of the isolated UCAM is substantially preserved for at least 45 days after initial procurement. In some embodiments, the biological and structural integrity of the isolated UCAM is substantially preserved for at least 50 days after initial procurement. In some embodiments, the biological and structural integrity of the isolated UCAM is substantially preserved for at least 55 days after initial procurement. In some embodiments, the biological and structural integrity of the isolated UCAM is substantially preserved for at least 60 days after initial procurement.

Further disclosed herein, in certain embodiments, a method of producing a tubular UCAM sheet, comprising: obtaining pre-frozen umbilical cord, and separating the UCAM from the umbilical vein and umbilical arteries and at least a portion of the Wharton's Jelly, wherein the structural integrity of the UCAM product is substantially preserved for at least 15 days after initial procurement. In some embodiments, the biological and structural integrity of the isolated UCAM is substantially preserved for at least 20 days after initial procurement. In some embodiments, the biological and structural integrity of the isolated UCAM is substantially preserved for at least 25 days after initial procurement. In some embodiments, the biological and structural integrity of the isolated UCAM is substantially preserved for at least 30 days after initial procurement. In some embodiments, the biological and structural integrity of the isolated UCAM is substantially preserved for at least 35 days after initial procurement. In some embodiments, the biological and structural integrity of the isolated UCAM is substantially preserved for at least 40 days after initial procurement. In some embodiments, the biological and structural integrity of the isolated UCAM is substantially preserved for at least 45 days after initial procurement. In some embodiments, the biological and structural integrity of the isolated UCAM is substantially preserved for at least 50 days after initial procurement. In some embodiments, the biological and structural integrity of the isolated UCAM is substantially preserved for at least 55 days after initial procurement. In some embodiments, the biological and structural integrity of the isolated UCAM is substantially preserved for at least 60 days after initial procurement.

Umbilical cord is recovered from any suitable source (e.g., a hospital or tissue bank). Umbilical cord can be obtained from any mammal, such as a human, non-human primate, cow or pig.

It is kept at -80°C until donor and specimen eligibility has been determined. In some embodiments, storing the umbilical cord at -80°C kills substantially all cells found in the umbilical cord. In some embodiments, storing the umbilical cord at -80°C kills substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM and the biological activity of the UCAM (e.g., its anti-inflammatory, anti-scarring, anti-antigenic, and anti-adhesion properties). In some embodiments, storing the umbilical cord at -80°C results in the loss of metabolic activity in substantially all cells found in the umbilical cord. In some embodiments, storing the umbilical cord at -80°C results in the loss of metabolic activity in substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM and the biological activity of the UCAM (e.g., its anti-inflammatory, anti-scarring, anti-antigenic, and anti-adhesion properties). In some embodiments, the umbilical cord is dried. In some embodiments, drying the umbilical cord kills substantially all cells found in the umbilical cord. In some embodiments, the umbilical cord is dried. In some embodiments, drying the umbilical cord results in the loss of metabolic activity in substantially all cells found in the umbilical cord.

### Initial Processing of UC

All processing is done following Good Tissue Practices (GTP) to ensure that no contaminants are introduced into the tissue grafts or UCAM.

The umbilical cord is tested for HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* using FDA licensed screening test. Any indication that the tissue is contaminated with HIV-1, HIV-2, HTLV-1, and hepatitis B and C results in the immediate quarantine and subsequent destruct of the tissue specimen.

Further, the donor's medical records are examined for risk factors for and clinical evidence of hepatitis B, hepatitis C, or HIV infection. Any indication that the donor has risk factors for, and/or clinical evidence of, infection with HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* results in the immediate quarantine and subsequent destruct of the tissue specimen.

The umbilical cord is frozen.

In some embodiments, substantially all of the blood is removed from the UC. In some embodiments, substantially all of the blood is removed from the umbilical cord before the umbilical cord is frozen. In some embodiments, blood is not removed from the UC. In some embodiments, blood is not removed from the umbilical cord before the umbilical cord is frozen.

In some embodiments, the umbilical cord tissue is washed with buffer to remove excess blood and tissue. In some embodiments, the umbilical cord is washed with an isotonic buffer. In some embodiments, the umbilical cord is washed with saline. In some embodiments, the umbilical cord is washed with PBS. In some embodiments, the umbilical cord is washed with PBS 1X. In some embodiments, the umbilical cord is washed with Ringer's solution. In some embodiments, the umbilical cord is washed with Hartmann's solution. In some embodiments, the umbilical cord is washed with a TRIS-buffered saline. In some embodiments, the umbilical cord is washed with a HEPES-buffered saline. In some embodiments, the umbilical cord is washed with EBSS. In some embodiments, the umbilical cord is washed with HBSS. In some embodiments, the umbilical cord is washed with Tyrode's Salt Solution. In some embodiments, the umbilical cord is washed with Gey's Balanced Salt Solution. In some embodiments, the umbilical cord is washed with DMEM. In some embodiments, the umbilical cord is washed with EMEM. In some embodiments, the umbilical cord is washed with GMEM. In some embodiments, the umbilical cord is washed with RPMI.

In some embodiments, the umbilical cord is washed with a buffer for 15 seconds to remove excess blood and tissue. In some embodiments, the umbilical cord is washed with a buffer for 30 seconds to remove excess blood and tissue. In some embodiments, the umbilical cord is washed with a buffer for 45 seconds to remove excess blood and tissue. In some embodiments, the umbilical cord is washed with a buffer for less than 1 minute to remove excess blood and tissue. In some embodiments, the umbilical cord is washed with a buffer for 1 minute to remove excess blood and tissue. In some embodiments, the umbilical cord is washed with a buffer for 2 minutes. In some embodiments, the umbilical cord is washed with a buffer for 3 minutes. In some embodiments, the umbilical cord is washed with a buffer for 4 minutes. In some embodiments, the umbilical cord is washed with a buffer for 5 minutes.

In some embodiments, the umbilical cord is cut into multiple tubular sections (e.g., using a scalpel). The size of each tubular sections depends on the desired use of the product (e.g., tissue graft) derived from the umbilical cord.

### Processing Method 1 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, a section of the umbilical cord is then cut longitudinally (e.g., using a scalpel or scissors). In some embodiments, the section of the umbilical cord is not cut into halves. In some embodiments, the section of the umbilical cord is cut into two halves. Optionally, in some embodiments, additional cuts are made in the Wharton's Jelly to help flatten out the UC.

In some embodiments, the cut umbilical cord tissue is optionally washed again with buffer to further remove excess blood and tissue.

In some embodiments, the umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape) In some embodiments, the umbilical cord is orientated such that the inside face of the umbilical cord (e.g., the face comprising the Wharton's Jelly) is facing up while the outside face (i.e., the face comprising UCAM) is facing the substrate. Optionally, in some embodiments, one end of the umbilical cord is left free (see FIGURE 3b). Alternatively, in some embodiments, both ends of the umbilical cord are left free.

If the umbilical cord does not lay flat against the substrate, in some embodiments, additional cuts are made in the Wharton's Jelly.

In some embodiments, part or all of the Wharton's Jelly is removed from the UCAM. The desired thickness of the tissue graft determines how much of the Wharton's Jelly is removed. In some embodiments, the Wharton's Jelly is peeled from the umbilical cord in layers (e.g., using a set of forceps, hemostats). In some embodiments, the Wharton's Jelly is cut away (e.g., shaved) from the umbilical cord in sections. In some embodiments, a rotoblator (i.e., a catheter attached to a drill with a diamond coated burr) is utilized to remove the Wharton's Jelly. In some embodiments, a liposuction machine is utilized to remove the Wharton's Jelly. In some embodiments, a liquid under high pressure is applied to remove the Wharton's Jelly. In some embodiments, a brush is utilized to remove the Wharton's Jelly (e.g., a mechanized brush rotating under high speed). In some embodiments, UCAM is retrieved using a surgical dermatome.

In some embodiments, both ends of the umbilical cord are attached to the substrate. In some embodiments, only one end is attached to the substrate. *See* FIGURE 3b. If one end of the umbilical cord is left free, in some embodiments, the free end of the umbilical cord is held (e.g., with a clamp, hemostats or a set of forceps (e.g., wide serrated tip forceps)) while part or all of the Wharton's Jelly is removed.

The umbilical cord comprises two arteries (the umbilical arteries) and one vein (the umbilical vein). In some embodiments, the vein and arteries are removed from the umbilical cord. In certain instances, the vein and arteries are surrounded (or suspended or buried) within the Wharton's Jelly. In some embodiments, the vein and arteries are removed concurrently with the removal of the Wharton's Jelly. In some embodiments, the vein and arteries are peeled (or pulled) from the umbilical cord (e.g., using a set of forceps). In some embodiments, the vein and arteries are cut away (e.g., shaved) from the umbilical cord in sections. In some embodiments, a rotoblator removes the vein and arteries concurrently with the Wharton's Jelly. In some embodiments, a liposuction machine is utilized to remove the vein and arteries concurrently with the Wharton's Jelly. In some embodiments, a vein stripper is utilized to remove the vein and arteries concurrently with the Wharton's Jelly. In some embodiments, a liquid under high pressure removes the vein and arteries concurrently with the Wharton's Jelly. In some embodiments, a brush removes the vein and arteries concurrently with the Wharton's Jelly. In some embodiments, a surgical dermatome removes the vein and arteries concurrently with the Wharton's Jelly.

After substantially pure UCAM has been obtained, the UCAM is optionally washed with buffer to remove excess blood and tissue.

### Processing Method 2 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, whole umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape). In some embodiments, UCAM is removed directly from the tubular UCAM. In some embodiments, UCAM is shaved off of the umbilical cord. In some embodiments, UCAM is shaved off of the umbilical cord using any suitable method. In some embodiments, UCAM is shaved off of the umbilical cord using a shaver (e.g., a cheese slicer) or a surgical dermatome.

After substantially pure UCAM has been obtained, the UCAM is optionally washed with buffer to remove excess blood and tissue.

### Processing to Generate Tubular UCAM Sheets

The size of the sheets depends on the desired use of the UCAM product.

In some embodiments, the UCAM sheets are contacted with a buffer to remove substantially all remaining red blood cells. In some embodiments, the UCAM sheets are contacted with an isotonic buffer. In some embodiments, the UCAM sheets are contacted with saline. In some embodiments, the UCAM sheets are contacted with PBS. In some embodiments, the UCAM sheets are contacted with PBS 1X. In some embodiments, the UCAM sheets are contacted with Ringer's solution. In some embodiments, the UCAM sheets are contacted with Hartmann's solution. In some embodiments, the UCAM sheets are contacted with a TRIS-buffered saline. In some embodiments, the UCAM sheets are contacted with a HEPES-buffered saline. In some embodiments, the UCAM sheets are contacted with EBSS. In some embodiments, the UCAM sheets are contacted with HBSS. In some embodiments, the UCAM sheets are contacted with Tyrode's Salt Solution. In some embodiments, the UCAM sheets are contacted with Gey's Balanced Salt Solution. In some embodiments, the UCAM sheets are contacted with DMEM. In some embodiments, the UCAM sheets are contacted with EMEM. In some embodiments, the UCAM sheets are contacted with GMEM. In some embodiments, the UCAM sheets are contacted with RPMI. In some embodiments, the UCAM sheets are contacted with EBSS.

In some embodiments, the UCAM sheets are contacted with a buffer to remove substantially all remaining red blood cells. In some embodiments, the UCAM sheets are contacted with a buffer for 10 minutes. In some embodiments, the UCAM sheets are contacted with a buffer for 15 minutes. In some embodiments, the UCAM sheets are contacted with a buffer for 20 minutes. In some embodiments, the UCAM sheets are contacted with a buffer for 25 minutes. In some embodiments, the UCAM sheets are contacted with a buffer for 30 minutes. In some embodiments, the UCAM sheets are contacted with a buffer for 35 minutes. In some embodiments, the UCAM sheets are contacted with a buffer for 40 minutes. In some embodiments, the UCAM sheets are contacted with a buffer for 45 minutes. In some embodiments, the UCAM sheets are contacted with a buffer for 50 minutes. In some embodiments, the UCAM sheets are contacted with a buffer for 55 minutes. In some embodiments, the UCAM sheets are contacted with a buffer for 60 minutes. In some embodiments, the UCAM sheets are contacted with a buffer for 2 hours. In some embodiments, the UCAM sheets are contacted with a buffer for 3 hours. In some embodiments, the UCAM sheets are contacted with a buffer for 4 hours. In some embodiments, the UCAM sheets are contacted with a buffer for 5 hours. In some embodiments, the UCAM sheets are contacted with a buffer for 6 hours. In some embodiments, the UCAM sheets are contacted with a buffer for 6 hours. In some embodiments, the UCAM sheets are contacted with a buffer for 10 hours. In some embodiments, the UCAM sheets are contacted with a buffer for 12 hours. In some embodiments, the UCAM sheets are contacted with a buffer for 18 hours. In some embodiments, the UCAM sheets are contacted with a buffer for 24 hours. In some embodiments, the UCAM sheets are contacted with a buffer for 2 days. In some embodiments, the UCAM sheets are contacted with a buffer for 3 days. In some embodiments, the UCAM sheets are contacted with a buffer for 4 days. In some embodiments, the UCAM sheets are contacted with a buffer for 5 days. In some embodiments, the UCAM sheets are contacted with a buffer for 6 days. In some embodiments, the UCAM sheets are contacted with a buffer for 7 days. In some embodiments, the buffer is optionally changed during the contacting (e.g., when the rate at which red blood cells diffuse from the UCAM sheets slows).

### Generation of Pulverized UCAM Product

Disclosed herein, in some embodiments, is a pulverized UCAM product, comprising: an isolated UCAM that does not comprise a cell with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum,* wherein the natural biological activity of the isolated UCAM is substantially preserved for at least 15 days after initial procurement. In some embodiments, the pulverized UCAM product does not comprise the umbilical veins or umbilical artery, or Wharton's Jelly. In some embodiments, the biological integrity of the isolated UCAM is substantially preserved for at least 20 days after initial procurement. In some embodiments, the biological integrity of the isolated UCAM is substantially preserved for at least 25 days after initial procurement. In some embodiments, the biological integrity of the isolated UCAM is substantially preserved for at least 30 days after initial procurement. In some embodiments, the biological integrity of the isolated UCAM is substantially preserved for at least 35 days after initial procurement. In some embodiments, the biological integrity of the isolated UCAM is substantially preserved for at least 40 days after initial procurement. In some embodiments, the biological integrity of the isolated UCAM is substantially preserved for at least 45 days after initial procurement. In some embodiments, the biological integrity of the isolated UCAM is substantially preserved for at least 50 days after initial procurement. In some embodiments, the biological integrity of the isolated UCAM is substantially preserved for at least 55 days after initial procurement. In some embodiments, the biological integrity of the isolated UCAM is substantially preserved for at least 60 days after initial procurement.

Further disclosed herein, in certain embodiments, a method of producing a pulverized UCAM product, comprising: obtaining pre-frozen umbilical cord, wherein the biological integrity of the UCAM product is substantially preserved for at least 15 days after initial procurement. In some embodiments, the method further comprises separating the UCAM from the umbilical vein and umbilical artery. In some embodiments, the method further comprises separating the UCAM from the Wharton's Jelly. In some embodiments, the biological integrity of the isolated UCAM is substantially preserved for at least 20 days after initial procurement. In some embodiments, the biological integrity of the isolated UCAM is substantially preserved for at least 25 days after initial procurement. In some embodiments, the biological integrity of the isolated UCAM is substantially preserved for at least 30 days after initial procurement. In some embodiments, the biological integrity of the isolated UCAM is substantially preserved for at least 35 days after initial procurement. In some embodiments, the biological integrity of the isolated UCAM is substantially preserved for at least 40 days after initial procurement. In some embodiments, the biological integrity of the isolated UCAM is substantially preserved for at least 45 days after initial procurement. In some embodiments, the biological integrity of the isolated UCAM is substantially preserved for at least 50 days after initial procurement. In some embodiments, the biological integrity of the isolated UCAM is substantially preserved for at least 55 days after initial procurement. In some embodiments, the biological integrity of the isolated UCAM is substantially preserved for at least 60 days after initial procurement.

Umbilical cord is recovered from any suitable source (e.g., a hospital or tissue bank). Umbilical cord can be obtained from any mammal, such as a human, non-human primate, cow or pig.

It is kept at -80°C until donor and specimen eligibility has been determined. In some embodiments, storing the umbilical cord at -80°C kills substantially all cells found in the umbilical cord. In some embodiments, storing the umbilical cord at -80°C kills substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM and the biological activity of the UCAM (e.g., its anti-inflammatory, anti-scarring, anti-antigenic, and anti-adhesion properties). In some embodiments, storing the umbilical cord at -80°C results in the loss of metabolic activity in substantially all cells found in the umbilical cord. In some embodiments, storing the umbilical cord at -80°C results in the loss of metabolic activity in substantially all cells found in the umbilical cord while maintaining or increasing the biological activity of the UCAM (e.g., its anti-inflammatory, anti-scarring, anti-antigenic, and anti-adhesion properties) relative to fresh (i.e., non-frozen) UCAM and the biological activity of the UCAM (e.g., its anti-inflammatory, anti-scarring, anti-antigenic, and anti-adhesion properties). In some embodiments, the umbilical cord is dried. In some embodiments, drying the umbilical cord kills substantially all cells found in the umbilical cord. In some embodiments, the umbilical cord is dried. In some embodiments, drying the umbilical cord results in the loss of metabolic activity in substantially all cells found in the umbilical cord.

### Initial Processing of UC

All processing is done following Good Tissue Practices (GTP) to ensure that no contaminants are introduced into the UCAM products.

The umbilical cord is tested for HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* using FDA licensed screening test. Any indication that the tissue is contaminated with HIV-1, HIV-2, HTLV-1, and hepatitis B and C results in the immediate quarantine and subsequent destruct of the tissue specimen.

Further, the donor's medical records are examined for risk factors for and clinical evidence of hepatitis B, hepatitis C, or HIV infection. Any indication that the donor has risk factors for, and/or clinical evidence of, infection with HIV-1, HIV-2, HTLV-1, hepatitis B and C, West Nile virus, cytomegalovirus, human transmissible spongiform encephalopathy (e.g., Creutzfeldt-Jakob disease) and *treponema pallidum* results in the immediate quarantine and subsequent destruct of the tissue specimen.

The umbilical cord is frozen.

In some embodiments, substantially all of the blood is removed from the UC. In some embodiments, substantially all of the blood is removed from the umbilical cord before the umbilical cord is frozen.

In some embodiments, the umbilical cord tissue is washed with buffer to remove excess blood and tissue.

In some embodiments, the umbilical cord is washed with an isotonic buffer or tissue culture media. In some embodiments, the umbilical cord is washed with saline. In some embodiments, the umbilical cord is washed with PBS. In some embodiments, the umbilical cord is washed with PBS 1X. In some embodiments, the umbilical cord is washed with a TRIS-buffered saline. In some embodiments, the umbilical cord is washed with a HEPES -buffered saline. In some embodiments, the umbilical cord is washed with Ringer's solution. In some embodiments, the umbilical cord is washed with Hartmann's solution. In some embodiments, the umbilical cord is washed with EBSS. In some embodiments, the umbilical cord is washed with HBSS. In some embodiments, the umbilical cord is washed with Tyrode's Salt Solution. In some embodiments, the umbilical cord is washed with Gey's Balanced Salt Solution. In some embodiments, the umbilical cord is washed with DMEM. In some embodiments, the umbilical cord is washed with EMEM. In some embodiments, the umbilical cord is washed with GMEM. In some embodiments, the umbilical cord is washed with RPMI.

In some embodiments, the umbilical cord is cut into multiple sections (e.g., using a scalpel). The size of the sections depends on the desired use of the product (e.g., UCAM product) derived from the umbilical cord.

### Processing Method 1 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, a section of the umbilical cord is then cut longitudinally (e.g., using a scalpel or scissors). In some embodiments, the section of the umbilical cord is not cut into halves. In some embodiments, the section of the umbilical cord is cut into two halves. Optionally, in some embodiments, additional cuts are made in the Wharton's Jelly to help flatten out the UC.

In some embodiments, the cut umbilical cord tissue is optionally washed again with buffer to further remove excess blood and tissue.

In some embodiments, the umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape) In some embodiments, the umbilical cord is orientated such that the inside face of the umbilical cord (e.g., the face comprising the Wharton's Jelly) is facing up while the outside face (i.e., the face comprising UCAM) is facing the substrate. Optionally, in some embodiments, one end of the umbilical cord is left free (see FIGURE 3b). Alternatively, in some embodiments, both ends of the umbilical cord are left free.

If the umbilical cord does not lay flat against the substrate, in some embodiments, additional cuts are made in the Wharton's Jelly.

In some embodiments, part or all of the Wharton's Jelly is removed from the UCAM. The desired thickness of the tissue graft determines how much of the Wharton's Jelly is removed. In some embodiments, the Wharton's Jelly is peeled from the umbilical cord in layers (e.g., using a set of forceps, hemostats). In some embodiments, the Wharton's Jelly is cut away (e.g., shaved) from the umbilical cord in sections. In some embodiments, a rotoblator (i.e., a catheter attached to a drill with a diamond coated burr) is utilized to remove the Wharton's Jelly. In some embodiments, a liposuction machine is utilized to remove the Wharton's Jelly. In some embodiments, a liquid under high pressure is applied to remove the Wharton's Jelly. In some embodiments, a brush is utilized to remove the Wharton's Jelly (e.g., a mechanized brush rotating under high speed). In some embodiments, UCAM is retrieved using a surgical dermatome.

In some embodiments, both ends of the umbilical cord are attached to the substrate. In some embodiments, only one end is attached to the substrate. *See* FIGURE 3b. If one end of the umbilical cord is left free, in some embodiments, the free end of the umbilical cord is held (e.g., with a clamp, hemostats or a set of forceps (e.g., wide serrated tip forceps)) while part or all of the Wharton's Jelly is removed.

The umbilical cord comprises two arteries (the umbilical arteries) and one vein (the umbilical vein). In some embodiments, the vein and arteries are removed from the umbilical cord. In certain instances, the vein and arteries are surrounded (or suspended or buried) within the Wharton's Jelly. In some embodiments, the vein and arteries are removed concurrently with the removal of the Wharton's Jelly. In some embodiments, the vein and arteries are peeled (or pulled) from the umbilical cord (e.g., using a set of forceps). In some embodiments, the vein and arteries are cut away (e.g., shaved) from the umbilical cord in sections. In some embodiments, a rotoblator removes the vein and arteries concurrently with the Wharton's Jelly. In some embodiments, a liposuction machine is utilized to remove the vein and arteries concurrently with the Wharton's Jelly. In some embodiments, a vein stripper is utilized to remove the vein and arteries concurrently with the Wharton's Jelly. In some embodiments, a liquid under high pressure removes the vein and arteries concurrently with the Wharton's Jelly. In some embodiments, a brush removes the vein and arteries concurrently with the Wharton's Jelly. In some embodiments, a surgical dermatome removes the vein and arteries concurrently with the Wharton's Jelly.

After substantially pure UCAM has been obtained, the UCAM is optionally washed with buffer to remove excess blood and tissue.

### Processing Method 2 to Isolate UCAM

In some embodiments, the umbilical cord is next contacted with a buffer to facilitate separation of the Wharton's Jelly and the UCAM. In some embodiments, the umbilical cord is contacted with an isotonic buffer or tissue culture media. In some embodiments, the umbilical cord is contacted with saline. In some embodiments, the umbilical cord is contacted with PBS. In some embodiments, the umbilical cord is contacted with PBS 1X. In some embodiments, the umbilical cord is contacted with Ringer's solution. In some embodiments, the umbilical cord is contacted with Hartmann's solution. In some embodiments, the umbilical cord is contacted with a TRIS-buffered saline. In some embodiments, the umbilical cord is contacted with a HEPES-buffered saline. In some embodiments, the umbilical cord is contacted with EBSS. In some embodiments, the umbilical cord is contacted with HBSS. In some embodiments, the umbilical cord is contacted with Tyrode's Salt Solution. In some embodiments, the umbilical cord is contacted with Gey's Balanced Salt Solution. In some embodiments, the umbilical cord is contacted with EMEM. In some embodiments, the umbilical cord is contacted with DMEM. In some embodiments, the umbilical cord is contacted with GMEM. In some embodiments, the umbilical cord is contacted with RPMI.

In some embodiments, whole umbilical cord is fastened onto a substrate (e.g., a styrofoam board) using any suitable method (e.g., it is fastened with needles or pins (e.g., T pins)). In some embodiments, the umbilical cord is stabilized with a substrate (e.g., absorbent towel cloth, drape). In some embodiments, UCAM is removed directly from the tubular UCAM. In some embodiments, UCAM is shaved off of the umbilical cord. In some embodiments, UCAM is shaved off of the umbilical cord using any suitable method. In some embodiments, UCAM is shaved off of the umbilical cord using a shaver (e.g., a cheese slicer) or a surgical dermatome.

After substantially pure UCAM has been obtained, the UCAM is optionally washed with buffer to remove excess blood and tissue.

### Processing to Generate Pulverized UCAM Product

In some embodiments the isolated UCAM is used to generate a pulverized UCAM product. As used herein, "pulverized UCAM product" means a UCAM product comprising tissue that has been broken up (or, disassociated). In some embodiments, the pulverized UCAM product is a homogenate. In some embodiments, the pulverized UCAM product is a dry powder. In some embodiments, the pulverized UCAM product is a solution, suspension or emulsion formed by mixing the UCAM powder with a carrier. In some embodiments, the pulverized UCAM product is formulated into a cream, lotion, ointment, paste, gel, film or paint.. In some embodiments, the pulverized UCAM product is contacted with a patch or wound dressing.

In some embodiments, the isolated UCAM is pulverized by any suitable method. In some embodiments, the isolated UMAM is pulverized by use of a pulverizer (e.g., a Bessman Tissue Pulverizer or a Covaris CryoPrep). In some embodiments, the isolated UCAM is pulverized by use of a tissue grinder (e.g., a Potter-Elvehjem grinder or a Wheaton Overhead Stirrer). In some embodiments, the isolated UCAM is pulverized by use of a sonicator. In some embodiments, the isolated UCAM is pulverized by use of a bead beater. In some embodiments, the isolated UMAM is pulverized by use of a freezer/mill (e.g., a SPEX SamplePrep Freezer/Mill). In some embodiments, the isolated UCAM is pulverized by use of a pestle and mortar. In some embodiments, the isolated UCAM is pulverized by manual use of a pestle and mortar.

In some embodiments, the isolated UCAM is optionally lyophilized before being pulverized. In some embodiments, the isolated UCAM is lyophilized by any suitable method (e.g., exposure to a liquid gas, placement in a freezer). In some embodiments, the isolated UCAM placed in the vacuum chamber of a lyophilization device until all or substantially all fluid (e.g., water) has been removed. In some embodiments, the isolated UCAM is lyophilized following freezing (e.g., exposure to a temperature below 0°C, -20°C, - 40°C, -50°C, -60°C, -70°C, -75°C, -80°C, -90°C, -100°C).

### Storage of UCAM and/or UCAM Products

In some embodiments, a UCAM product or a UCAM product disclosed herein is stored for later use. In some embodiments, storing a UCAM product or a UCAM product disclosed herein does not destroy the integrity of the UCAM extracellular matrix. In some embodiments, the UCAM product is pulverized. In some embodiments, the UCAM or a UCAM product is lyophilized. In some embodiments, the UCAM or the UCAM product is stored in any suitable storage medium. In some embodiments, the UCAM or the UCAM product is stored in 50% DMEM + 50% Glycerol. In some embodiments, the UCAM or the UCAM product is stored in 10%, 20%, 30%, 40%, 50%, 60%, 70%, 80%, 90% or 100% glycerol. In some embodiments, the UCAM or the UCAM product is stored in10%, 20%, 30%, 40%, 50%, 60%, 70%, 80%, 90% or 100% propylene glycol.

In some embodiments, the UCAM or a UCAM product is optionally contacted with a substrate (i.e., a supportive backing). In some embodiments, the UCAM is not contacted with a substrate. In some embodiments, the UCAM or a UCAM product is orientated such that the UCAM is in contact with the substrate. In some embodiments, the UCAM or a UCAM product is orientated such that the stroma is in contact with the substrate. In some embodiments, the UCAM or a UCAM product is orientated such that the epithelial side is in contact with the substrate.

In some embodiments, the isolated UCAM or a UCAM product is attached to the substrate. In some embodiments, the substrate is nitrocellulose paper (NC). In some embodiments, the substrate is nylon membrane (NM). In some embodiments, the substrate is polyethersulfone membrane (PES).

### Cryopreservation

In some embodiments, the isolated UCAM or UCAM product is frozen for cryopreservation. In some embodiments, cryopreserving a UCAM product or a UCAM product disclosed herein does not destroy the integrity of the UCAM extracellular matrix. In some embodiments, the isolated UCAM or UCAM product is exposed to a liquid gas (e.g., liquid nitrogen or liquid hydrogen). In some embodiments, the isolated UCAM or UCAM product is exposed to liquid nitrogen. In some embodiments, the isolated UCAM or UCAM product does not contact the liquid gas. In some embodiments, the isolated UCAM or UCAM product is placed in a container and the container is contacted with liquid gas. In some embodiments, the isolated UCAM or UCAM product is exposed to the liquid gas until the UCAM product or UCAM is frozen.

### Lyophilization

In some embodiments, the isolated UCAM or UCAM product is lyophilized. In some embodiments, the isolated UCAM or UCAM product is lyophilized following freezing. In some embodiments, the isolated UCAM or UCAM product is lyophilized following freezing by any suitable method (e.g., exposure to a liquid gas, placement in a freezer). In some embodiments, the isolated UCAM or UCAM product is frozen by exposure to a temperature below about 0°C. In some embodiments, the isolated UCAM or UCAM product is frozen by exposure to a temperature below about -20°C. In some embodiments, the isolated UCAM or UCAM product is frozen by exposure to a temperature below about - 40°C. In some embodiments, the isolated UCAM or UCAM product is frozen by exposure to a temperature below about -50°C. In some embodiments, the isolated UCAM or UCAM product is frozen by exposure to a temperature below about -60°C. In some embodiments, the isolated UCAM or UCAM product is frozen by exposure to a temperature below about - 70°C. In some embodiments, the isolated UCAM or UCAM product is frozen by exposure to a temperature below about -75°C. In some embodiments, the isolated UCAM or UCAM product is frozen by exposure to a temperature below about -80°C. In some embodiments, the isolated UCAM or UCAM product is frozen by exposure to a temperature below about - 90°C. In some embodiments, the isolated UCAM or UCAM product is frozen by exposure to a temperature below about -100°C. In some embodiments, the isolated UCAM or UCAM product is frozen by exposure to a liquid gas.

In some embodiments, the cryopreserved isolated UCAM or UCAM product is lyophilized. In some embodiments, the cryopreserved isolated UCAM or UCAM product is placed in the vacuum chamber of a lyophilization device until all or substantially all fluid (e.g., water) has been removed.

### Sterilization

In some embodiments, a UCAM product disclosed herein is subject to terminal sterilization by any suitable (e.g., medically acceptable) method. In some embodiments, the lyophilized UCAM or UCAM product is exposed to gamma radiation for a period of time sufficient to sterilize the isolated UCAM or UCAM product. In some embodiments, the lyophilized UCAM or UCAM product is exposed to gamma radiation at 25 kGy for a period of time sufficient to sterilize the isolated UCAM or UCAM product. In some embodiments, the lyophilized UCAM or UCAM product is exposed to an electron beam for a period of time sufficient to sterilize the UCAM or UCAM product. In some embodiments, the lyophilized UCAM or UCAM product is exposed to X-ray radiation for a period of time sufficient to sterilize the UCAM or UCAM product. In some embodiments, the lyophilized isolated membrane or UCAM product is exposed to UV radiation for a period of time sufficient to sterilize the UCAM or UCAM product.

### Rehydration

In some embodiments, the UCAM or UCAM product is partially or fully rehydrated. In some embodiments, the isolated UCAM or UCAM product is rehydrated by contacting the isolated UCAM or UCAM product with a buffer or with water. In some embodiments, the UCAM is contacted with an isotonic buffer. In some embodiments, the UCAM is contacted with saline. In some embodiments, the UCAM is contacted with PBS. In some embodiments, the UCAM is contacted with Ringer's solution. In some embodiments, the UCAM is contacted with Hartmann's solution. In some embodiments, the UCAM is contacted with a TRIS-buffered saline. In some embodiments, the UCAM is contacted with a HEPES-buffered saline; 50% DMEM + 50% Glycerol; 10%, 20%, 30%, 40%, 50%, 60%, 70%, 80%, 90% or 100% glycerol; and/or 10%, 20%, 30%, 40%, 50%, 60%, 70%, 80%, 90% or 100% propylene glycol..

In some embodiments, the UCAM is contacted with a buffer for 10 minutes. In some embodiments, the UCAM is contacted with a buffer for 15 minutes. In some embodiments, the UCAM is contacted with a buffer for 20 minutes. In some embodiments, the UCAM is contacted with a buffer for 25 minutes. In some embodiments, the UCAM is contacted with a buffer for 30 minutes. In some embodiments, the UCAM is contacted with a buffer for 35 minutes. In some embodiments, the UCAM is contacted with a buffer for 40 minutes. In some embodiments, the UCAM is contacted with a buffer for 45 minutes. In some embodiments, the UCAM is contacted with a buffer for 50 minutes. In some embodiments, the UCAM is contacted with a buffer for 55 minutes. In some embodiments, the UCAM is contacted with a buffer for 60 minutes. In some embodiments, the UCAM is contacted with a buffer for 2 hours. In some embodiments, the UCAM is contacted with a buffer for 3 hours. In some embodiments, the UCAM is contacted with a buffer for 4 hours. In some embodiments, the UCAM is contacted with a buffer for 5 hours. In some embodiments, the UCAM is contacted with a buffer for 6 hours. In some embodiments, the UCAM is contacted with a buffer for 6 hours. In some embodiments, the UCAM is contacted with a buffer for 10 hours. In some embodiments, the UCAM is contacted with a buffer for 12 hours. In some embodiments, the UCAM is contacted with a buffer for 18 hours. In some embodiments, the UCAM is contacted with a buffer for 24 hours.

### UCAM Product Formulations

In some embodiments, pulverized UCAM is a solution, suspension or emulsion formed by mixing pulverized UCAM with a carrier. In some embodiments, the pulverized UCAM is a homogenate. In some embodiments, the pulverized UCAM is a powder. In some embodiments, the pulverized UCAM is a reconstituted powder. In some embodiments, the UCAM product is formulated for topical administration.

Pharmaceutical topical formulations disclosed herein are formulated in any suitable manner. Any suitable technique, carrier, and/or excipient is contemplated for use with the LPA receptor antagonists disclosed herein.

### Creams and Lotions

Disclosed herein, in certain embodiments, is a topical formulation of a UCAM product wherein the topical formulation is in the form of a cream. In certain instances, creams are semisolid (e.g., soft solid or thick liquid) formulations that include a UCAM product dispersed in an oil-in-water emulsion or a water-in-oil emulsion.

Disclosed herein, in certain embodiments, is a topical formulation of a UCAM product wherein the topical formulation is in the form of a lotion. In certain instances, lotions are fluid emulsions (e.g., oil-in-water emulsions or a water-in-oil emulsion). In some embodiments, the hydrophobic component of a lotion and/or cream is derived from an animal (e.g., lanolin, cod liver oil, and ambergris), plant (e.g., safflower oil, castor oil, coconut oil, cottonseed oil, menhaden oil, palm kernel oil, palm oil, peanut oil, soybean oil, rapeseed oil, linseed oil, rice bran oil, pine oil, sesame oil, or sunflower seed oil), or petroleum (e.g., mineral oil, or petroleum jelly).

### Ointments

Disclosed herein, in certain embodiments, is a topical formulation of a UCAM product wherein the topical formulation is in the form of an ointment. In certain instances, ointments are semisolid preparations that soften or melt at body temperature.

### Pastes

Disclosed herein, in certain embodiments, is a topical formulation of a UCAM product wherein the topical formulation is in the form of a paste. In certain instances, pastes contain at least 20% solids. In certain instances, pastes are ointments that do not flow at body temperature.

### Gels and Films

Disclosed herein, in certain embodiments, is a topical formulation of a UCAM product wherein the topical formulation is in the form of a gel. In certain instances, gels are semisolid (or semi-rigid) systems consisting of dispersions of large organic molecules dispersed in a liquid. In certain instances, gels are water-soluble and are removed using warm water or saline.

In certain instances, in the treatment of dermal lesions, contacting lesions with a dressing can often disturb injured tissues. The removal of many dressings for wounds such as burns surface lesions that involve a significant area of the skin can cause significant pain and often can re-open at least portions of partially healed wounds. In some instances, the topical formulations described herein are applied as a liquid to the affected area and the liquid gels as a film on the affected area. In some instances the film is a water soluble film and can be removed with water or a mild aqueous detergent, avoiding pain and discomfort associated with the removal of wound dressings. In certain instances, the topical formulation described herein is a dermal film comprising a flexible film made of a polyalkyloxazoline. In some instances, the film has a structural layer made of a polyalkyloxazoline and a pressure sensitive adhesive layer that keeps the film in place.

### Sticks

Disclosed herein, in certain embodiments, is a topical formulation of a UCAM product wherein the topical formulation is in the form of a stick. In certain instances, sticks are solid dosage forms that melt at body temperature. In some embodiments, a stick comprises a wax, a polymer, a resin, dry solids fused into a firm mass, and/or fused crystals. In some embodiments, a topical formulation of a UCAM product is in the form of a styptic pencil (i.e., a stick prepared by (1) heating crystals until they lose their water of crystallization and become molten, and (2) pouring the molten crystals into molds and allowing them to harden). In some embodiments, a topical formulation of a UCAM product is in the form of stick wherein the stick comprises a wax (e.g., the wax is melted and poured into appropriate molds in which they solidify in stick form).

In some embodiments, a topical formulation of a UCAM product is in the form of stick wherein the stick comprises a melting base (i.e., a base that softens at body temperature). Examples of melting bases include, but are not limited to, waxes, oils, polymers and gels. In some embodiments, a topical formulation of a UCAM product is in the form of stick wherein the stick comprises a moisten base (i.e., a base that is activated by the addition of moisture).

### Patches

Disclosed herein, in certain embodiments, is a topical formulation of a UCAM product wherein the topical formulation is administered via a patch. In some embodiments, a topical UCAM formulation disclosed herein is dissolved and/or dispersed in a polymer or an adhesive. In some embodiments, a film, a patch disclosed herein is constructed for continuous, pulsatile, or on demand delivery of a UCAM product.

### Wound Dressings

Disclosed herein, in certain embodiments, is a topical formulation of a UCAM product wherein the topical formulation is administered with (or via) a wound dressing. Wound dressings include, but are not limited to gauzes, transparent film dressings, hydrogels, polyurethane foam dressings, hydrocolloids and alginates. In certain instances, wound dressings promote wound healing. In some instances wound dressings reduce or inhibit aberrant wound healing.

### Miscellaneous Formulations

In some embodiments, the formulations and compositions disclosed herein are administered as a dermal paint. As used herein, paints (also known as film formers) are solutions comprised of a solvent, a monomer or polymer, an active agent, and optionally one or more pharmaceutically-acceptable excipients. After application to a tissue, the solvent evaporates leaving behind a thin coating comprised of the monomers or polymers, and the active agent. The coating protects active agents and maintains them in an immobilized state at the site of application. This decreases the amount of active agent which may be lost and correspondingly increases the amount delivered to the affected area of the skin of an individual. By way of non-limiting example, paints include collodions (e.g. Flexible Collodion, USP), and solutions comprising saccharide siloxane copolymers and a cross-linking agent. Collodions are ethyl ether/ethanol solutions containing pyroxylin (a nitrocellulose). After application, the ethyl ether/ethanol solution evaporates leaving behind a thin film of pyroxylin. In solutions comprising saccharide siloxane copolymers, the saccharide siloxane copolymers form the coating after evaporation of the solvent initiates the cross-linking of the saccharide siloxane copolymers.

In certain embodiments, the topical formulations described herein comprise UCAM products that are optionally incorporated within controlled release particles, lipid complexes, liposomes, nanoparticles, microspheres, microparticles, nanocapsules or other agents which enhance or facilitate localized delivery to the skin. An example of a conventional microencapsulation process for pharmaceutical preparations is shown in U.S. Pat. No. 3,737,337.

In some instances, a topical formulation described herein is a liposomal formulation. Liposomes are prepared by introducing an aqueous buffer into a mixture of phospholipid and organic solvent and the organic solvent is subsequently removed by evaporation under reduced pressure. An example of a liposomal preparation is described in Proc. Natl. Acad. Sci. 1978, 75, 4194-98. Liposomes are fractionated according to their particle sizes by size exclusion chromatography (SEC). The subfractions of liposomes are further sized by photon correlation spectroscopy (PCS) for their particle sizes. Enzymatic assays (e.g., phosphatidylcholine (PC) assay) are used to analyze lipid contents of liposomes.

### Dermatological Excipients

Disclosed herein, in certain embodiments, is a topical formulation of a UCAM product wherein the topical formulation comprises a carrier. Suitable carriers include water, hyaluronan, collagen, ethanol, polyols (propyleneglycol, polyethylene-glycol, glycerol, cremophor and the like), vegetable oils (such as olive oil), injectable organic esters (e.g., ethyl oleate), fatty oils (e.g., sesame oil), and synthetic fatty acid esters (e.g., ethyl oleate or triglycerides).

### Penetration Enhancers

Disclosed herein, in certain embodiments, is a topical formulation of a UCAM product wherein the topical formulation comprises a penetration enhancer. Penetration enhancers include, but are not limited to, sodium lauryl sulfate, sodium laurate, polyoxyethylene-20-cetyl ether, laureth-9, sodium dodecylsulfate, dioctyl sodium sulfosuccinate, polyoxyethylene-9-lauryl ether (PLE), Tween 80, nonylphenoxypolyethylene (NP-POE), polysorbates, sodium glycocholate, sodium deoxycholate, sodium taurocholate, sodium taurodihydrofusidate, sodium glycodihydrofusidate, oleic acid, caprylic acid, mono- and di-glycerides, lauric acids, acylcholines, caprylic acids, acylcarnitines, sodium caprates, EDTA, citric acid, salicylates, DMSO, decylmethyl sulfoxide, ethanol, isopropanol, propylene glycol, polyethylene glycol, glycerol, propanediol, and diethylene glycol monoethyl ether. In certain embodiments, the topical formulations described herein are designed for minimal systemic exposure and include, for example, low amounts of penetration enhancers.

### Gelling Agents

Disclosed herein, in certain embodiments, is a topical formulation of a UCAM product wherein the topical formulation comprises a gelling (or thickening) agent. In some embodiments, a topical formulation disclosed herein further comprises from about 0.1% to about 5%, from about 0.1% to about 3%, or from about 0.25% to about 2%, of a gelling agent. In certain embodiments, the viscosity of a topical formulation disclosed herein is in the range from about 100 to about 500,000 cP, about 100 cP to about 1,000 cP, about 500 cP to about 1500 cP, about 1000 cP to about 3000 cP, about 2000 cP to about 8,000 cP, about 4,000 cP to about 10,000 cP, about 10,000 cP to about 50,000 cP. Suitable gelling agents for use in preparation of the gel topical formulation include, but are not limited to, celluloses, cellulose derivatives, cellulose ethers (e.g., carboxymethylcellulose, ethylcellulose, hydroxyethylcellulose, hydroxymethylcellulose, hydroxypropylmethylcellulose, hydroxypropylcellulose, methylcellulose), guar gum, xanthan gum, locust bean gum, alginates (e.g., alginic acid), silicates, starch, tragacanth, carboxyvinyl polymers, carrageenan, paraffin, petrolatum, acacia (gum arabic), agar, aluminum magnesium silicate, sodium alginate, sodium stearate, bladderwrack, bentonite, carbomer, carrageenan, carbopol, xanthan, cellulose, microcrystalline cellulose (MCC), ceratonia, chondrus, dextrose, furcellaran, gelatin, ghatti gum, guar gum, hectorite, lactose, sucrose, maltodextrin, mannitol, sorbitol, honey, maize starch, wheat starch, rice starch, potato starch, gelatin, sterculia gum, polyethylene glycol (e.g. PEG 200-4500), gum tragacanth, ethyl cellulose, ethylhydroxyethyl cellulose, ethylmethyl cellulose, methyl cellulose, hydroxyethyl cellulose, hydroxyethylmethyl cellulose, hydroxypropyl cellulose, poly(hydroxyethyl methacrylate), oxypolygelatin, pectin, polygeline, povidone, propylene carbonate, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl-cellulose (HPMC), sodium carboxymethyl-cellulose (CMC), silicon dioxide, polyvinylpyrrolidone (PVP: povidone), or combinations thereof.

Gels include a single-phase or a two-phase system. A single-phase gel consists of organic macromolecules distributed uniformly throughout a liquid in such a manner that no apparent boundaries exist between the dispersed macromolecules and the liquid. Some single-phase gels are prepared from synthetic macromolecules (e.g., carbomer) or from natural gums, (e.g., tragacanth). In some embodiments, single-phase gels are generally aqueous, but will also be made using alcohols and oils. Two-phase gels consist of a network of small discrete particles.

Gels can also be classified as being hydrophobic or hydrophilic. In certain embodiments, the base of a hydrophobic gel consists of a liquid paraffin with polyethylene or fatty oils gelled with colloidal silica, or aluminum or zinc soaps. In contrast, the base of hydrophobic gels usually consists of water, glycerol, or propylene glycol gelled with a suitable gelling agent (e.g., tragacanth, starch, cellulose derivatives, carboxyvinylpolymers, and magnesium-aluminum silicates).

Suitable agents for use in formulations that are applied as liquids and gel upon application to the skin into a film include but are not limited to polymers composed of polyoxypropylene and polyoxyethylene that are known to form thermoreversible gels when incorporated into aqueous solutions. These polymers have the ability to change from the liquid state to the gel state at temperatures close to body temperature, therefore allowing useful formulations that are applied as gels and/or films to the affected area. Examples of polymers that gel at body temperature and are used in gels and/or films described herein include and are not limited to poloxamers (e.g., PLURONICS F68®, F88®, F108®, and F127®, which are block copolymers of ethylene oxide and propylene oxide). The liquid state-to-gel state phase transition is dependent on the polymer concentration and the ingredients in the solution.

### Adhesives

In some instances, the topical formulations described herein comprise pressure sensitive adhesives (e.g., polyalkyloxazoline polymers) and allow for application of an adhesive film to an affected area of skin.

### Emollients

Disclosed herein, in certain embodiments, is a topical formulation of a UCAM product wherein the topical formulation comprises an emollient. Emollients include, but are not limited to, castor oil esters, cocoa butter esters, safflower oil esters, cottonseed oil esters, corn oil esters, olive oil esters, cod liver oil esters, almond oil esters, avocado oil esters, palm oil esters, sesame oil esters, squalene esters, kikui oil esters, soybean oil esters, acetylated monoglycerides, ethoxylated glyceryl monostearate, hexyl laurate, isohexyl laurate, isohexyl palmitate, isopropyl palmitate, methyl palmitate, decyloleate, isodecyl oleate, hexadecyl stearate decyl stearate, isopropyl isostearate, methyl isostearate, diisopropyl adipate, diisohexyl adipate, dihexyldecyl adipate, diisopropyl sebacate, lauryl lactate, myristyl lactate, and cetyl lactate, oleyl myristate, oleyl stearate, and oleyl oleate, pelargonic acid, lauric acid, myristic acid, palmitic acid, stearic acid, isostearic acid, hydroxystearic acid, oleic acid, linoleic acid, ricinoleic acid, arachidic acid, behenic acid, erucic acid, lauryl alcohol, myristyl alcohol, cetyl alcohol, hexadecyl alcohol, stearyl alcohol, isostearyl alcohol, hydroxystearyl alcohol, oleyl alcohol, ricinoleyl alcohol, behenyl alcohol, erucyl alcohol, 2-octyl dodecanyl alcohol, lanolin and lanolin derivatives, beeswax, spermaceti, myristyl myristate, stearyl stearate, carnauba wax, candelilla wax, lecithin, and cholesterol.

### Miscellaneous Excipients

In certain embodiments, a topical formulation comprising a UCAM product comprises additional excipients such as, by way of example, abrasives, absorbents, anticaking agents, astringents, essential oils, fragrances, skin-conditioning agents, skin healing agents, skin protectants (e.g., sunscreens, or ultraviolet light absorbers or scattering agents), skin soothing agents, or combinations thereof.

### Methods of Use

Disclosed herein, in certain embodiments, are methods of using a UCAM product disclosed herein.

The scope of the invention is defined by the claims. Any references in the description to methods of treatment refer to the compounds, pharmaceutical compositions and medicaments of the present invention for use in a method for treatment of the human (or animal) body by therapy (or for diagnosis).

In some embodiments, a UCAM product disclosed herein is used to inhibit at least one of the following: scarring, inflammation, adhesion and angiogenesis. In some embodiments, a UCAM product disclosed herein is used to promote wound healing. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product is used as a covering (e.g., a wound covering). In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product is used to promote wound repair. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product is used as a barrier to adhesion. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, the UCAM product comprises UCAM as a scaffold, and a plurality of cells integrated into the UCAM scaffold. In some embodiments, the cells are embryonic stem cells, mesenchymal stem cells or adult lineage-committed stem cells or differentiated epidermal cells (e.g., to treat a burn or a surgical incision in the skin). In some embodiments, the cells are mesothelial cells (e.g., to treat to a wound (e.g., surgical incision) in an internal organ).

### Injured Tissue Repair and Supplementation

Disclosed herein, in certain embodiments, is the use of a UCAM product for repairing, reconstructing, replacing, or supplementing a recipient's damaged, compromised, or missing tissue. In some embodiments, the UCAM product is used as a wound covering or is used to facilitate wound repair. In some embodiments, the use is a homologous use (e.g., a functional homologous use or a structural homologous use). In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the tissue was damaged, compromised, or lost due to an injury (e.g., a burn; a surgical incision; an area of necrosis resulting from an infection, trauma, or a toxin; a laceration). In some embodiments, the tissue was damaged, compromised, or lost due to a burn. In some embodiments, the tissue was damaged, compromised, or lost due to a wound (e.g., an incision, laceration, abrasion). In some embodiments, the tissue was damaged, compromised, or lost due to necrosis. In some embodiments, the tissue was damaged, compromised, or lost due to ulceration.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, the UCAM product comprises UCAM as a scaffold, and a plurality of cells integrated into the UCAM scaffold. In some embodiments, the cells are epidermal cells (e.g., to treat a burn or a surgical incision in the skin). In some embodiments, the cells are mesothelial cells (e.g., to treat to a wound (e.g., surgical incision) in an internal organ).

### Burns

In some embodiments, a UCAM product or UCAM product disclosed herein is used as a protective graft over a burn. In some embodiments, a UCAM product or UCAM product disclosed herein is used as a protective graft over a first degree burn. In some embodiments, a UCAM product or UCAM product disclosed herein is used as a protective graft over a second degree burn. In some embodiments, a UCAM product or UCAM product disclosed herein is used as a protective graft over a third degree burn. In some embodiments, a protective graft comprising an UCAM or tissue graft as described herein is placed on a burn.

In some embodiments, the protective graft comprises UCAM. In some embodiments, the protective graft comprises UCAM as a scaffold, and a plurality of epidermal cells integrated into the UCAM scaffold.

### Wounds

In some embodiments, a UCAM product or UCAM product disclosed herein is used as a protective graft over a wound in the skin (e.g., an incision, laceration, abrasion, ulcer, puncture, penetration).

In some embodiments, a protective graft comprising an UCAM or tissue graft as described herein is placed on wound. In some embodiments, the protective graft comprises UCAM. In some embodiments, the protective graft comprises UCAM as a scaffold, and a plurality of epithelial cells (e.g., epidermal and/or mesothelial cells) integrated into the UCAM scaffold.

In some embodiments, a UCAM product or UCAM product disclosed herein is used as a covering over an incision in an organ (e.g., the skin, brain, stomach, kidneys, liver, intestines, lungs, bladder, trachea, esophagus, vagina, ureter, and blood vessel walls). In some embodiments, a UCAM product or UCAM product disclosed herein is placed on a surgical incision. In some embodiments, a UCAM product disclosed herein is used to repair or supplement tissue following colon resection. In some embodiments, a UCAM product disclosed herein is used to repair or supplement tissue following gastrectomy. In some embodiments, a UCAM product disclosed herein is used to repair or supplement tissue following breast surgery (e.g., breast reduction surgery, breast augmentation surgery, and mastectomy). In some embodiments, UCAM product or UCAM product disclosed herein comprises UCAM as a scaffold, and a plurality of epithelial cells (e.g., epidermal and/or mesothelial cells) integrated into the UCAM scaffold.

In some embodiments, a UCAM product or UCAM product disclosed herein is used as a covering over an incision in the skin (e.g., an incision to the epidermis, dermis, and/or hypodermis). In some embodiments, a UCAM product disclosed herein is used to repair or supplement the skin following hemorrhoid surgery.

### Necrosis

In some embodiments, a UCAM product or UCAM product disclosed herein is used as a protective graft over an area of necrotic tissue (e.g., from an infection). In some embodiments, a UCAM product or UCAM product disclosed herein is used as a protective graft over an area of necrotic skin. In some embodiments, a protective graft comprising an UCAM or tissue graft as described herein is placed on an area of necrotic tissue. In some embodiments, the protective graft comprises UCAM. In some embodiments, the protective graft comprises UCAM as a scaffold, and a plurality of epidermal and/or mesothelial cells integrated into the UCAM scaffold.

### Ulcer

In some embodiments, a UCAM product or UCAM product disclosed herein is used as a protective covering over an ulcer. In some embodiments, the protective covering comprises UCAM as a scaffold, and a plurality of epidermal and/or mesothelial cells integrated into the UCAM scaffold. In some embodiments, a protective covering is placed on an ulcer.

In some embodiments, the ulcer is a foot ulcer (e.g., a diabetic foot ulcer or an arterial insufficiency ulcer). In some embodiments, treating a foot ulcer comprises (a) preparing the wound (e.g., debriding the wound); and (b) placing a UCAM product or UCAM product disclosed herein on the wound. In some embodiments, treating a foot ulcer comprises (a) preparing the wound (e.g., debriding the wound); (b) placing a UCAM product or UCAM product disclosed herein on the wound; and (c) covering the UCAM product or UCAM product with a protective barrier (e.g., a silvercell dressing, metipel, gauze, or a bandage).

In some embodiments, the ulcer is a venous stasis (VS) ulcer. In some embodiments, treating a VS ulcer comprises (a) preparing the wound (e.g., debriding the wound); and (b) placing a UCAM product or UCAM product disclosed herein on the wound. In some embodiments, treating a VS ulcer comprises (a) preparing the wound (e.g., debriding the wound); (b) placing a UCAM product or UCAM product disclosed herein on the wound; and (c) covering the UCAM product or UCAM product with a protective barrier (e.g., a silvercell dressing, metipel, gauze, or a bandage).

In some embodiments, the ulcer is a corneal ulcer (i.e., ulcerative keratitis). In some embodiments, treating a corneal ulcer comprises (a) preparing the wound (e.g., debriding the wound); and (b) placing a UCAM product or UCAM product disclosed herein on the wound. In some embodiments, treating a corneal ulcer comprises (a) preparing the wound (e.g., debriding the wound); (b) placing a UCAM product or UCAM product disclosed herein on the wound; and (c) covering the UCAM product or UCAM product with a protective barrier (e.g., a contact lens or a bandage).

### Soft Tissue Uses

Disclosed herein, in certain embodiments, is the use of an UCAM product for repairing, reconstructing, replacing, or supplementing a recipient's damaged, compromised, or missing soft tissue (e.g., tendons). In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, the UCAM product comprises UCAM as a scaffold, and a plurality of cells integrated into the UCAM scaffold. In some embodiments, the cells are epidermal cells (e.g., to treat a burn or a surgical incision in the skin). In some embodiments, the cells are mesothelial cells (e.g., to treat to a wound (e.g., surgical incision) in an internal organ).

In some embodiments, a UCAM product described herein is used as a covering over an incision in soft tissue (e.g., eyelids form the tissue plane between different layers of soft tissue).

In some embodiments, a UCAM product or UCAM product disclosed herein is used as structural (tectonic) support for soft tissue.

In some embodiments, a UCAM product or UCAM product disclosed herein prevents adhesion in joint or tendon repairs.

In some embodiments, a UCAM product or UCAM product disclosed herein is used in the repair a tendon or joint (such as rotator cuff repairs, hand tendon repairs). In some embodiments, a UCAM product or UCAM product disclosed herein is used as a patch over a tendon (e.g., a tendon that has been torn or a tendon that has been sutured) or joint. In some embodiments, a UCAM product or UCAM product disclosed herein is used to reconstruct a tendon. In some embodiments, a UCAM product or UCAM product disclosed herein is used to augment a tendon or joint. In some embodiments, a UCAM product or UCAM product disclosed herein is used to reinforce a tendon or joint. In some embodiments, a UCAM product or UCAM product disclosed herein is used to prevent adhesion of a healing tendon to surrounding tissue, tendons or joints. In some embodiments, a UCAM product or UCAM product disclosed herein is used to prevent the formation of scar tissue on a tendon.

In some embodiments, a UCAM product disclosed herein is used to augment smaller tendons and ligaments of the foot and ankle, including the posterior tibial tendon, the personneal tendons, the flexor and extensor tendons, and the ligaments of the lateral ankle complex. In some embodiments, a UCAM product disclosed herein is used to reinforce primary repair of the quadriceps and patellar tendons surrounding the knee. In some embodiments, a UCAM product disclosed herein is used as a periosteal patch for bone graft in joint replacement.. In some embodiments, a UCAM product disclosed herein is used augment deficient hip and knee capsular tissue following total joint revision surgery.

In some embodiments, a UCAM product or UCAM product disclosed herein is used in the repair of a torn rotator cuff. In some embodiments, a UCAM product or UCAM product disclosed herein is used as a patch over a rotator cuff muscle or tendon (e.g., the supraspinatus tendon). In some embodiments, a UCAM product or UCAM product disclosed herein is used to reconstruct a rotator cuff muscle or tendon (e.g., the supraspinatus tendon). In some embodiments, a UCAM product or UCAM product disclosed herein is used to augment a rotator cuff muscle or tendon (e.g., the supraspinatus tendon). In some embodiments, a UCAM product or UCAM product disclosed herein is used to reinforce a rotator cuff muscle or tendon (e.g., the supraspinatus tendon). In some embodiments, a UCAM product or UCAM product disclosed herein is used to prevent adhesion of soft tissue to a rotator cuff muscle or tendon (e.g., the supraspinatus tendon).

In some embodiments, a UCAM product or UCAM product disclosed herein is used in the repair gingiva. In some embodiments, a UCAM product or UCAM product disclosed herein is used in the repair gingival recession. In some embodiments, a UCAM product or UCAM product disclosed herein is used as a patch over gingiva. In some embodiments, a UCAM product or UCAM product disclosed herein is used as a patch over an exposed tooth root surface. In some embodiments, a UCAM product or UCAM product disclosed herein is used to reconstruct gingiva. In some embodiments, a UCAM product or UCAM product disclosed herein is used to augment gingiva. In some embodiments, a UCAM product or UCAM product disclosed herein is used to reinforce gingiva. In some embodiments, a UCAM product or UCAM product disclosed herein is used to prevent adhesion of soft tissue to gingiva.

In some embodiments, a UCAM product described herein is used as a protective graft over an incision or tear in the fascia. In some embodiments, a UCAM product or UCAM product disclosed herein is used as structural (tectonic) support the fascia. In some embodiments, a UCAM product or UCAM product disclosed herein is used as a replacement or supplement for the fascia. In some embodiments, a UCAM product or UCAM product disclosed herein is used to repair a hernia (e.g., to repair the fascia). In some embodiments, a UCAM product or UCAM product disclosed herein is used to repair an inguinal hernia. In some embodiments, a UCAM product or UCAM product disclosed herein is used to repair a femoral hernia. In some embodiments, a UCAM product or UCAM product disclosed herein is used to repair an umbilical hernia. In some embodiments, a UCAM product or UCAM product disclosed herein is used to repair an incisional hernia. In some embodiments, a UCAM product or UCAM product disclosed herein is used to repair a diaphragmatic hernia. In some embodiments, a UCAM product or UCAM product disclosed herein is used to repair a Cooper's hernia, an epigastric hernia, an hiatal hernia, a Littre's hernia, a lumbar hernia, a maydl hernia, an obturator hernia, a pantaloon hernia, a paraesophageal hernia, a paraumbilical hernia, a perineal hernia, a properitoneal hernia, a Richter's hernia, a sliding hernia, a sciatic hernia, a spigelian hernia, a sports hernia, a Velpeau hernia, or a Amyand's hernia.

In some embodiments, a UCAM product or UCAM product disclosed herein is used to repair a spinal disc herniation. In some embodiments, a UCAM product described herein is used as a protective graft over an incision or tear in a spinal disc. In some embodiments, a UCAM product described herein is used as a protective graft over an incision or tear in an annulus fibrosis. In some embodiments, a UCAM product or UCAM product disclosed herein is used as structural (tectonic) support a spinal disc. In some embodiments, a UCAM product or UCAM product disclosed herein is used as structural (tectonic) support an annulus fibrosis. In some embodiments, a UCAM product or UCAM product disclosed herein is used as a replacement or supplement for a spinal disc. In some embodiments, a UCAM product or UCAM product disclosed herein is used as structural (tectonic) support a spinal disc. In some embodiments, a UCAM product or UCAM product disclosed herein is used as a replacement or supplement for an annulus fibrosis.

In some embodiments, a UCAM product or UCAM product disclosed herein is used as a protective graft over an incision in the brain, or in one (or all) of the meninges (i.e., the dura mater, the pia mater, and/or the arachnoid mater). In some embodiments, a UCAM product or UCAM product disclosed herein is used as structural (tectonic) support for one (or all) of the meninges (i.e., the dura mater, the pia mater, and/or the arachnoid mater). In some embodiments, a UCAM product or UCAM product disclosed herein is used as a replacement for one (or all) of the meninges (i.e., the dura mater, the pia mater, and/or the arachnoid mater).

In some embodiments, a UCAM product or UCAM product disclosed herein is used as a protective graft over an incision in a lung or in the pleura. In some embodiments, a UCAM product or UCAM product disclosed herein is used as structural (tectonic) support for the pleura. In some embodiments, a UCAM product or UCAM product disclosed herein is used as a replacement for the pleura.

In some embodiments, a UCAM product or UCAM product disclosed herein is used as a protective graft over an incision in a tympanic membrane. In some embodiments, a UCAM product or UCAM product disclosed herein is used as structural (tectonic) support for a tympanic membrane. In some embodiments, a UCAM product or UCAM product disclosed herein is used as a replacement for a tympanic membrane.

In some embodiments, a UCAM product or UCAM product disclosed herein is used as a protective graft over an incision in the heart or the pericardium. In some embodiments, a UCAM product or UCAM product disclosed herein is used as structural (tectonic) support for the pericardium. In some embodiments, a UCAM product or UCAM product disclosed herein is used as a replacement for the pericardium.

In some embodiments, a UCAM product or UCAM product disclosed herein is used as a protective graft over an incision in the peritoneum. In some embodiments, a UCAM product or UCAM product disclosed herein is used as structural (tectonic) support for the peritoneum. In some embodiments, a UCAM product or UCAM product disclosed herein is used as a replacement for the peritoneum.

### Ophthalmic Uses

Disclosed herein, in certain embodiments, is the use of a UCAM product for repairing, reconstructing, replacing, or supplementing a recipient's damaged, compromised, or missing ocular tissue. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, the UCAM product comprises UCAM as a scaffold, and a plurality of cells integrated into the UCAM scaffold.

### Treatment of Glaucoma

As used herein, "Glaucoma" means a disorder characterized by the loss of retinal ganglion cells in the optic nerve. In certain instances, glaucoma partially or fully results from an increase in intraocular pressure in the anterior chamber (AC). Intraocular pressure varies depending on the production of liquid aqueous humor by the ciliary processes of the eye and the drainage of the aqueous humor through the trabecular meshwork.

Glaucoma Drainage Devices (GDD) are medical devices that are implanted into an eye to relieve intraoccular pressure by providing an alternative pathway for the aqueous humor to drain. If left uncovered, a GDD tube will erode and leave the eye susceptible to intraocular infection. Thus, the GDD tube needs to be covered. Currently, patches used to cover GDD tubes are made from pericardium, sclera and cornea. These patches are about 400-550 microns thick. The thinness of these patches results in their melting by 25% in 2 years potentially leaving the shunt tube exposed again.

Disclosed herein, is the use of a UCAM product or UCAM product disclosed herein as a patch to cover GDD tubes. In some embodiments, the tissue graft comprises UCAM. In some embodiments, a UCAM product or UCAM product disclosed herein is 300-600 microns thick. In some embodiments, a UCAM product or UCAM product disclosed herein does not melt by 25% in 2 years.

### Treatment of Ocular Ulcers

Disclosed herein, is the use of a UCAM product or UCAM product disclosed herein as a patch to cover persistent epithelial defects and/or ulcers in eyes.

In some embodiments the base of the ulcer is debrided with surgical sponges and the poorly adherent epithelium adjacent to the edge of the ulcer is removed (e.g., to the section of the eye where the epithelium becomes quite adherent). In some embodiments, the UCAM product or UCAM product is transferred to the recipient eye, with the stromal surface facing the eye and fitted to cover the defect by trimming off the excess edges of the UCAM product or UCAM product. In some embodiments, the UCAM product or UCAM product is then secured to the eye by sutures (e.g., interrupted 10-0 nylon sutures or running 10-0 nylon sutures) with the suture knots being buried. In some embodiments, the UCAM product or UCAM product is secured to the eye by use of fibrin glue. In some embodiments, a protective layer is applied over the UCAM product or UCAM product or the entire eye (e.g., a contact lens). In some embodiments, an antibiotic is applied to the UCAM product or UCAM product or the entire eye (e.g., neomycin, polymyxin b sulfate and dexamethasone).

### Conjunctival, scleral, lid, and orbital rim surface reconstruction

Disclosed herein, in certain embodiments, is the use of a UCAM product or UCAM product disclosed herein in conjunctival, scleral, lid, and orbital rim surface reconstruction. In some embodiments, damage to the conjunctival surface results from symblepharon lysis; surgical removal of tumor, lesion, and/or scar tissue; excimer laser photorefractive keratectomy and therapeutic keratectomy; or combinations thereof.

### Cell Transplant

Disclosed herein, in some embodiments, is the use of a UCAM product or UCAM product disclosed herein as a scaffold for transplanting a plurality of retinal cells to a host. In some embodiments, the retinal cells are transplanted to a retina. In some embodiments, at least one hole is formed in a retina. In some embodiments, a retina is partially detached. In some embodiments, the scaffold and cells to be transplanted are placed on the target site.

### Coronary Uses

Disclosed herein, in certain embodiments, is the use of a UCAM product for repairing, reconstructing, replacing, or supplementing a recipient's damaged, compromised, or missing coronary tissue. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, the UCAM product comprises UCAM as a scaffold, and a plurality of cells integrated into the UCAM scaffold. In some embodiments, the cells are mesothelial cells (e.g., to treat to a wound (e.g., surgical incision) in an internal organ).

### Coronary Artery Bypass

Disclosed herein, is the use of a UCAM product described herein in coronary artery bypass surgery. In some embodiments, an isolated tubular UCAM product is grafted onto a coronary artery to bypass a section of the artery that is characterized by atherosclerosis. In some embodiments, the UCAM product comprises UCAM. In some embodiments, the UCAM product or UCAM product described herein is cultured with a plurality of fibroblasts and endothelial cells.

### Heart Valves

In some embodiments, a UCAM product or UCAM product disclosed herein is used as a protective graft over a heart valve. In some embodiments, a UCAM product or UCAM product disclosed herein is used as structural (tectonic) support for a heart valve. In some embodiments, a UCAM product or UCAM product disclosed herein is used as a replacement for a heart valve.

### Veins and Arteries

In some embodiments, a UCAM product or UCAM product disclosed herein is used as a protective covering over a vein or artery. In some embodiments, a UCAM product or UCAM product disclosed herein is used as structural (tectonic) support for a vein or artery. In some embodiments, a UCAM product or UCAM product disclosed herein is used as a replacement for vein or artery.

### Nerve Uses

Disclosed herein, in certain embodiments, is the use of a UCAM product for repairing, reconstructing, replacing, or supplementing a recipient's damaged, compromised, or missing nerve. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, a UCAM product described herein is used as a covering over a nerve (e.g., a peripheral nerve). In some embodiments, a UCAM product described herein is used as a covering over a nerve graft, nerve transfer, or a repaired nerve. In some embodiments, a UCAM product described herein is used as a covering over an incision in a nerve (e.g., a peripheral nerve). In some embodiments, a UCAM product or UCAM product disclosed herein is used as structural (tectonic) support for a nerve (e.g., a peripheral nerve). In some embodiments, a UCAM product or UCAM product disclosed herein prevents adhesion in nerve repair.

In some embodiments, a UCAM product described herein is used as a non-constricting encasement for injured nerves. In some embodiments, a UCAM product described herein prevents or minimizes scar formation, encapsulation, chronic compression, tethering of a nerve, and nerve entrapment. In some embodiments, a UCAM product described herein prevents or minimizes neuroma formation. In some embodiments, a UCAM product described herein prevents or minimizes the migration of endogenous growth factors (i.e. Nerve Growth Factor) present during nerve repair.

### Spinal Uses

Disclosed herein, in certain embodiments, is the use of a UCAM product described herein during spinal surgery. In some embodiments, a UCAM product described herein during a laminectomy. In some embodiments, the use is a homologous use. In some embodiments, the UCAM product is minimally manipulated. In some embodiments, the UCAM product does not comprise another article, except for water, crystalloids, or a sterilizing, preserving, or storage agent. In some embodiments, the UCAM product does not have a systemic effect and is not dependent upon the metabolic activity of living cells for its primary function.

In some embodiments, the UCAM product comprises UCAM. In certain instances, the UCAM comprises proteins, glycans, protein-glycan complexes (e.g., a complex of hyaluronic acid and a heavy chain of |α| and PTX3) and enzymes that promote tissue repair. For example, the stroma of UCAM contains growth factors, anti-angiogenic and anti-inflammatory proteins, as well as natural inhibitors to various proteases. In some embodiments, proteins and enzymes found in the UCAM diffuse out of the UCAM and into the surrounding tissue.

In some embodiments, a UCAM product described herein is used to reduce or prevent epidural fibrosis and/or scar adhesions following spinal surgery (e.g., laminectomy). In some embodiments, a UCAM product described herein is implanted between dura mater and overlying tissue following spinal surgery (e.g., laminectomy). In some embodiments, implanting a UCAM product described herein between dura mater and overlying tissue following spinal surgery (e.g., laminectomy) reduces or prevents migration of fibroblasts to the dura mater and collagen deposition on the dura mater.

In some embodiments, a UCAM product described herein is used to reduce or prevent the development of proliferative scarring following spinal surgery (e.g., laminectomy). In some embodiments, a UCAM product described herein is used to reduce or prevent the development of a postoperative (e.g., postlaminectomy) epidural/peridural/perineural scar. In some embodiments, a UCAM product described herein is used to reduce or prevent the development of proliferative scarring following spinal surgery (e.g., laminectomy). In some embodiments, a UCAM product disclosed herein is used to reduce or prevent the development of a postlaminectomy membrane.

In some embodiments, a UCAM product described herein is used to reduce or prevent the development of extradural compression or dural teethering following spinal surgery (e.g., laminectomy). In some embodiments, a UCAM product described herein is used to reduce or prevent the development of tethered nerve roots following spinal surgery (e.g., laminectomy). In some embodiments, a UCAM product described herein is used to reduce or prevent the development of arachnoiditis following spinal surgery (e.g., laminectomy).

In some embodiments, a UCAM product disclosed herein comprises UCAM as a scaffold, and a tissue integrated into the UCAM scaffold. In some embodiments, the tissue is morcelized bone tissue. In some embodiments, a UCAM product comprising UCAM as a scaffold and tissue integrated into the UCAM scaffold (e.g., morcelized bone tissue) is used during a spinal fusion procedure. In some embodiments, a UCAM product comprising UCAM as a scaffold and tissue integrated into the UCAM scaffold (e.g., morcelized bone tissue) is implanted between adjacent vertebrae. In some embodiments, implantation of a UCAM product comprising UCAM as a scaffold and tissue integrated into the UCAM scaffold (e.g., morcelized bone tissue) between two adjacent vertebrae promotes fusion of the vertebrae.

In some embodiments, a UCAM product or UCAM product disclosed herein is used as a protective graft over an incision in the dura mater. In some embodiments, a UCAM product or UCAM product disclosed herein is used as structural (tectonic) support for the dura mater. In some embodiments, a UCAM product or UCAM product disclosed herein is used as a replacement for the dura mater.

### Uses for Pulverized UCAM product

Disclosed herein, in certain instances, is a pulverized UCAM product comprising UCAM. In some embodiments, the pulverized UCAM is a homogenate. In some embodiments, the pulverized UCAM product is a dry powder. In some embodiments, the pulverized UCAM product is a reconstituted dry power. In some embodiments, the UCAM product further comprises a carrier. In some embodiments, the carrier is water. In some embodiments, the carrier is saline. In some embodiments, the carrier is hyaluronic acid. In some embodiments, the carrier is collagen. In some embodiments, the pulverized UCAM product is formulated into a cream, lotion, ointment, paste, gel, film, or paint. In some embodiments, the pulverized UCAM product is applied to a patch or wound dressing.

In some embodiments, a pulverized UCAM product disclosed herein is used as a dermal filler. In some embodiments, a pulverized UCAM product disclosed herein is injected into subdermal facial tissues. In some embodiments, a pulverized UCAM product disclosed herein is injected under wrinkles and aging lines of the face (e.g., nasolabial folds, melomental folds, "crow's feet" and forehead wrinkles). In some embodiments, a pulverized UCAM product disclosed herein is used for lip augmentation. In some embodiments, a pulverized UCAM product disclosed herein is injected into the lips.

In some embodiments, a pulverized UCAM product disclosed herein is used to treat arthritis (e.g., osteoarthritis, rheumatoid arthritis, septic arthritis, ankylosing spondylitis, spondylosis). In some embodiments, a pulverized UCAM product disclosed herein is injected into an arthritic joint (e.g., a knee).

### Cell Culture

Disclosed herein, in some embodiments, is the use of UCAM as disclosed herein as a scaffold for culturing a plurality of cells (e.g., embryonic stem cells, mesenchymal stem cells, induced pluripotent stem cells). In some embodiments, an UCAM as described herein is used as a scaffold for culturing a plurality of keratocytes. In some embodiments, an UCAM as described herein is used as a scaffold for culturing a plurality of fibroblasts. In some embodiments, an UCAM as described herein is used as a scaffold for culturing a plurality of retinal pigment epithelial (RPE) cells. In some embodiments, an UCAM as described herein is used as a scaffold for culturing a plurality of epithelial cells. In some embodiments, an UCAM as described herein is used as a scaffold for culturing a plurality of epithelial stem cells. In some embodiments, an UCAM as described herein is used as a scaffold for culturing a plurality of limbal stem cells.

In some embodiments, at least one cell is contacted with isolated UCAM as disclosed herein. In some embodiments, the cell is cultured on isolated UCAM as disclosed herein under conditions suitable for growth for a period of time sufficient to produce a plurality of cells.

### EXAMPLES

### Example 1 - Isolation of UCAM from UC

An umbilical cord was obtained. The umbilical cord was washed with PBS 1X to remove any excess tissue or blood.

Using a scalpel, the umbilical cord was cut into 5 cm long sections and each section was cut longitudinally without sectioning it in half. Next, the umbilical cord was washed with PBS 1X and suspended in the buffer for 30 minutes.

The umbilical cord was then fastened down onto a flat styrofoam board with syringe needle tips. The umbilical cord was oriented such that the cut face faced upwards while the UCAM faced the board. A scalpel was used to make cuts into the Wharton's Jelly to flatten the tubular UC. A 0.12 forcep was used to pull out arteries/vein from the surrounding stromal tissue of the UC. The umbilical cord was then washed off with 1X PBS.

### Example 2 - Optimization of RBC Removal from UCAM

UCAM was kept in 50% DMEM + 50% glycerol in -80°C freezer for 2 weeks after processing. Upon thawing, the UCAM& preservation solution was inspected and kept in - 4°C for 2 days. After that the UCAMs were subjected to the treatment to try to remove the red blood cells according to the following instructions:

| **Media** | **Duration** | **Temperature(°C)** | **Cosmetic Observation** |
|---|---|---|---|
| 50% DMEM + 50% glycerol | 2 days | 4°C | Improved UCAM clarity. Significant removal of red blood cells from tissue as evidence by the preservation media turning red. |
| 50% DMEM + 50% glycerol | 4 days | 4°C | Complete removal of red blood cells. UCAM is completely clear. Preservation media was changed at Day 2. |
| 2% Acetic Acid | 15 minutes | Room temperature (22 °C) | AM tissue turned slightly brown after 15 minutes of immersion. UCAM feels stiffer after treatment. |
| 1% Hydrochloric Acid | 15 minutes | Room temperature (22 °C) | AM tissue turned brown possibly due to the red blood cells hemolyzing. Cosmetically, it made the tissue looked worst. |

Conclusion: 50% DMEM + 50% glycerol preservation solution provides the best cosmetic outcome to remove red blood cells from UCAM

### Example 3 - Optimization of RBC Removal from UCAM

The temperature is set at 4°C since our prior validation study shows that cryopreserved UCAM has a shelf life of 3 months at this temperature.

We tested three UCAM sizes; small (s) - 1 x 2 cm; medium (m) - 5.3 x 4 cm; and large (I) - 5.3 x 8 cm.

Preservation media was changed at Day 1 (t=24h), Day 3 (t=72h) and Day 6 (t=144h). UCAM washed with PBS 1X prior to change of media.

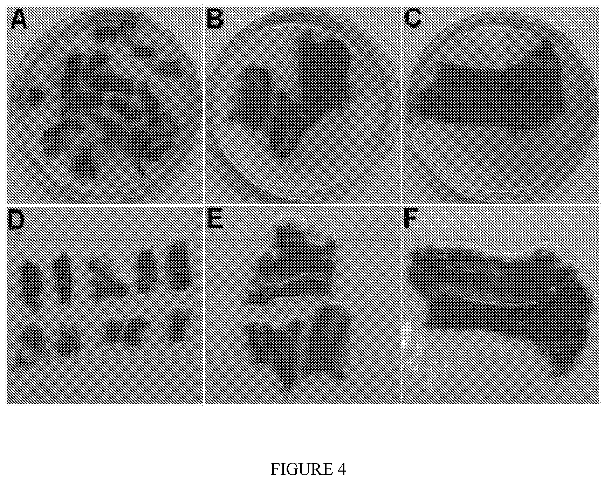

From FIGURE 4, it is apparent that the UCAM cleared up significantly after being kept in 50% DMEM + 50% Glycerol at 4°C for 7 days. We can also conclude that the size of the tissue affects the amount of red blood cells being removed within a specified time. The tissues that are cut into smaller pieces (1cm x 2cm) showed significant cosmetic improvement followed by the medium tissues (5.3 cm x 4cm) and finally the large tissue (5.3cm x 8cm) which showed the least cosmetic improvement.

### Example 4 - Optimization of RBC Removal from UCAM

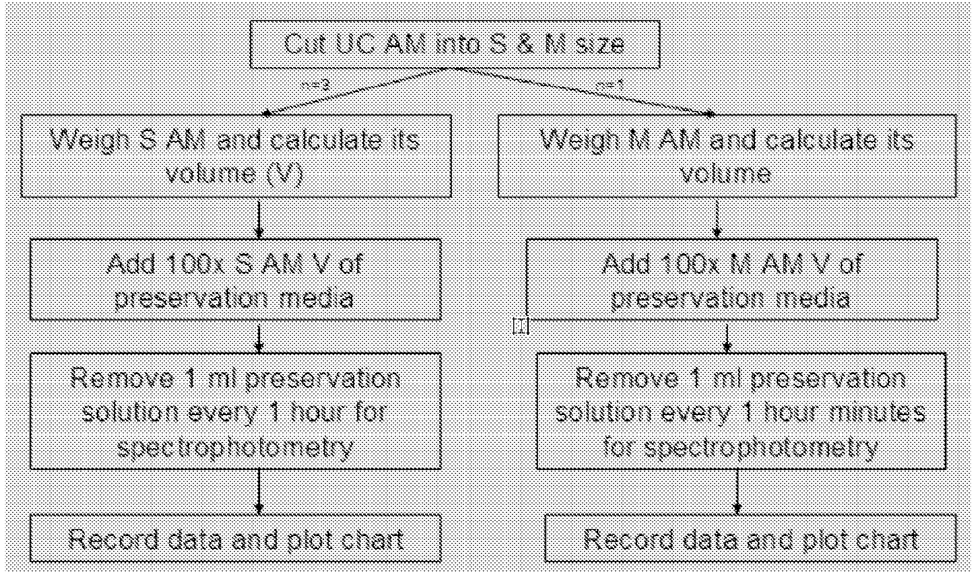

The rate of RBC removal was determined by measuring the absorbance of the preservation media.

The UCAM was cut into two UCAM sizes: small (S) - 1.0 x 0.75 cm and medium (M) - 3.5 x 3.5 cm.

The weight of the UCAM was determined and the volume calculated.

Preservation media was added to 100x the volume of the UCAM.

| **Size** | **Weight (g)** | | **Volume (mL)** |
|---|---|---|---|
| S | 0.187 | Average = 0.200 | 60 |
| | 0.217 | | |
| | 0.197 | | |
| M | 1.55 | | 155 |

1 mL of preservation media was removed every hour for spectrophotometry.

The absorbance data collected did not provide any information as it was too small even when we used the Micro-Bradley Method. Reading obtained ranged from 0 to 0.050 A. This shows that the 100X volume dilution is too much as the red blood cells concentration was not even detected by the photometer. This data is confirmed by visual inspection of the preservation media as no detectable coloration was observed. However, visual inspection of the UCAM showed that red blood cells were removed. The S sized UCAM showed significant removal of the red blood cells compared to the M sized UCAM proving the size of the UCAM plays a role in the rate of red blood cells removal.

Smaller UCAM showed significant red blood cells reduction compared to medium sized UCAM. No data was obtained on the duration till equilibrium is reached as the 100X volume dilution is too large to detect the trace amounts of red blood cells. As such, no media change was required and the red blood cells for the S UCAM cleared up after 5 days.

### Example 5 - Optimization of RBC Removal from UCAM

Six sections of UCAM are cut into 1.0 X 0.75 mm size samples.

The UCAM is placed in a 250 mL bottle with preservation media (50% DMEM + 50% Glycerol) and incubated at 4°C.

The preservation media is changed every four hours until the media remain clear.

### Example 6 - Optimization of RBC Removal from UCAM

The protocol for small UCAM according to Example 7 was used with one modification - a magnetic stirrer was added to the preservation media.

Our initial results indicated that the duration for red blood cell removal is shortened to 3 days instead of the usual 5 days if the whole system is agitated with a magnetic stirrer.

### Example 7 - Isolation of UCAM from UC

An umbilical cord was obtained. The cord blood was removed the UC. The umbilical cord was washed with PBS 1X to remove any excess tissue or blood.

Using a scalpel, the umbilical cord was cut into 5 cm sections. Each of the umbilical cord sections was cut longitudinally without sectioning it in half. Additional cuts were made in the Wharton's Jelly to flatten out each section. Next, the sections were washed with PBS 1X three times to remove excess blood and tissue.

Next, the sections were suspended for 30 minutes is PBS 1X to facilitate the separation of the Wharton's Jelly and the UCAM.

The umbilical cord was then fastened down onto a flat styrofoam board with syringe needle tips. The umbilical cord was oriented such that the cut face faced upwards while the UCAM faced the board. *See* FIGURE 3b.

Wide serrated tip forceps were used to hold the UCAM at the free end of the UC. At the same time, a pointed serrated tip forceps was used to slowly peel off the Wharton's Jelly. There were several layers of tissue with the final clear tissue being the UCAM.

Once the UCAM layer was identified, any remaining Wharton's Jelly was slowly removed using pointed serrated tip forceps while using the wide serrated tip forceps as a clamp. The Wharton's Jelly was separable from the UCAM as a sheet but some force was required. The arteries and vein separated from the UCAM together with the Wharton's Jelly.

The UCAM was triple in PBS 1X to remove excess blood and tissue.

Once a satisfactory UCAM was obtained, it was cut it into sections measuring 1.0 x 0.75 cm and again washed with PBS 1X.

### Example 8 - Lyophilization of UCAM

Isolated UCAM prepared according to Example 2 was used.

The UCAM was adhered with its sticky side up (epithelium side down) onto a piece of Nylon Membrane (NM). The UCAM/NM was placed in a 60 mm culture dish.

The UCAM was frozen by holding the dish on the surface of liquid nitrogen. Caution was taken to ensure that the liquid nitrogen did not overflow into the dish and come in contact with the UCAM/NM.

The frozen UCAM/NM was lyophilized by placing it in a vacuum chamber of a lyophilization machine for 20 hours.

Once lyophilization was complete, the UCAM was detached from the NM by slowly peeling it off. It was then placed it in a pouch for storage and exposed to gamma radiation at 25 kGy.

### Example 9 - Optimization of Lyophilization

The individual UCAM was measured before lyophilization (after being suspended in PBS 1X for 12h), after lyophilization and after rehydration. The visual clarity of each tissue after each of these steps was also determined.

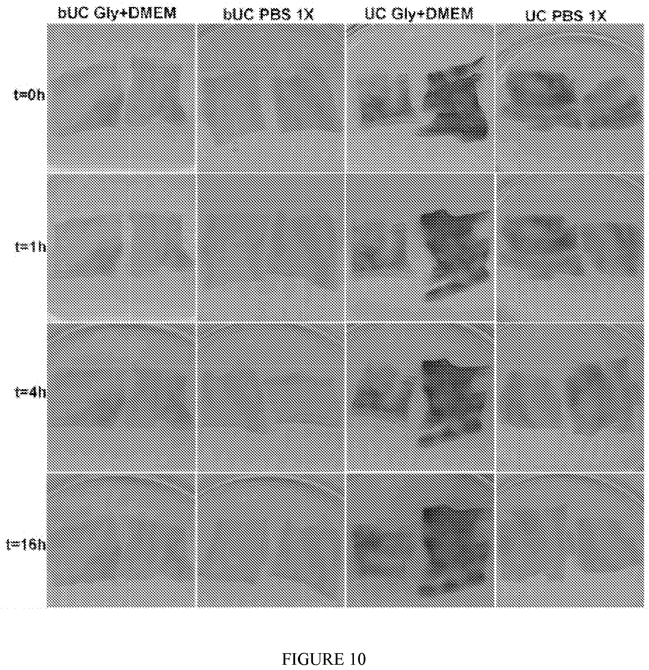

The UCAM lost an average weight lost of 93% after lyophilization. The freeze dried UCAM regained an average of 32% of its original weight after being reconstituted in 50% DMEM + 50% Glycerol for 1 hour. *See* FIGURE 10.

| **Size** | **Weight** | | | **Manipulation** | | |
|---|---|---|---|---|---|---|
| | **BL** | **AL** | **AR** | **BL** | **AL** | **AR** |
| Medium | 2.440 | 0.147 | 0.806 | Opaque, elastic, agile, easily folded, thick | Clear but with brown regions (dried red blood cells), sticky, easily crumpled, extremely thin | Regained some of its opaqueness and elasticity, not as sticky but tissue is still folded, thicker than AL but not as thick as BL |
| Small 1 | 0.331 | 0.013 | 0.155 | | | |
| Small 2 | 0.729 | 0.058 | 0.214 | | | |
| Small 3 | 0.363 | 0.025 | 0.149 | | | |
| Small 4 | 0.445 | 0.032 | 0.206 | | | |
| Small 5 | 0.535 | 0.035 | 0.187 | | | |

### Example 10 - Optimization of Lyophilization

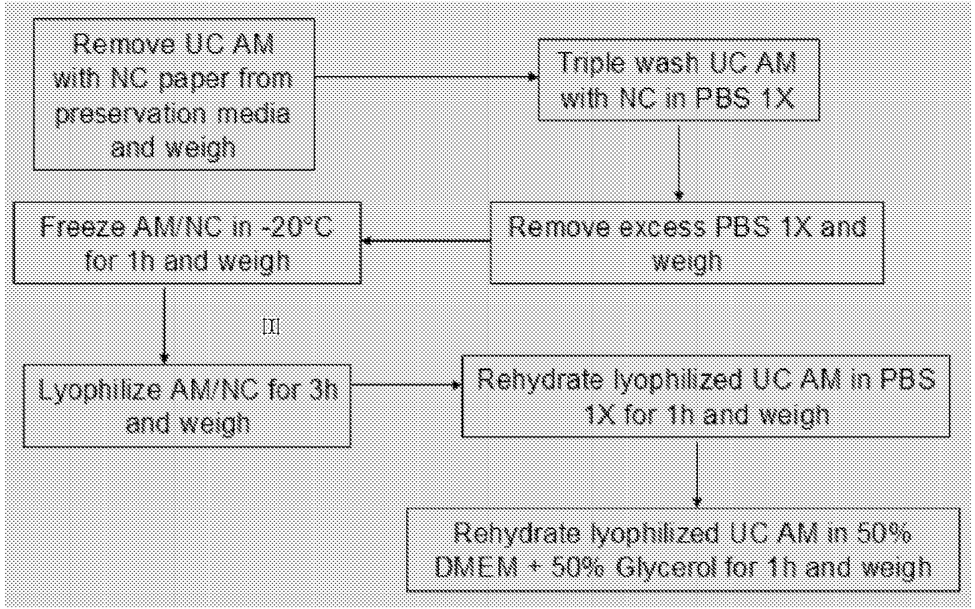

The UCAM lost 75% of its weight after lyophilization. However it gained back 58% of its weight after rehydration in PBS 1X for an hour.

| **Size** | **Weight with NC Paper** | | | | |
|---|---|---|---|---|---|
| | BL (GLY) | BL (PBS) | AL | AR (PBS) | AR (GLY) |
| S1 | 0.356 | 0.331 | 0.083 | 0.166 | 0.187 |
| S2 | 0.324 | 0.317 | 0.065 | 0.137 | 0.184 |
| S3 | 0.258 | 0.258 | 0.06 | 0.141 | 0.17 |
| S4 | 0.304 | 0.339 | 0.079 | 0.163 | 0.17 |
| M | 1.78 | 2.04 | 0.553 | 1.293 | 0.942 |

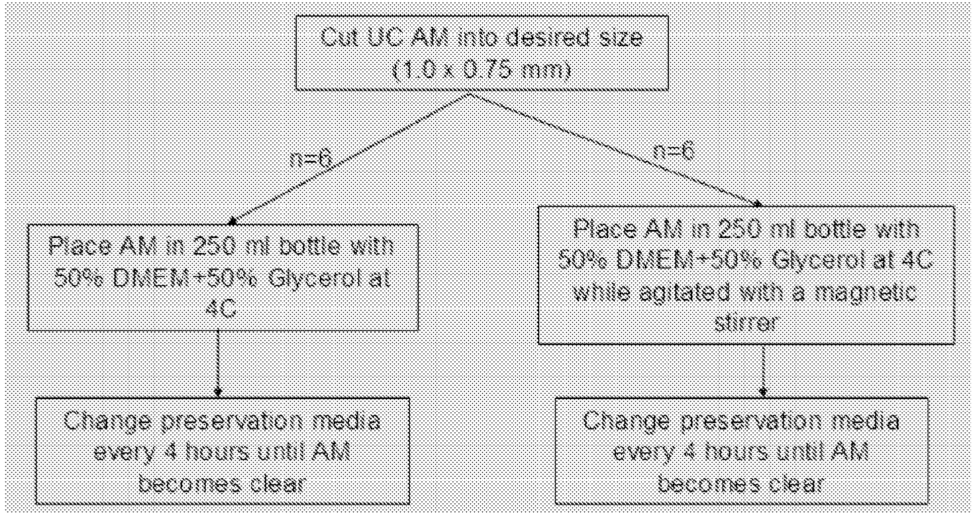

### Example 11 - Optimization of RBC Removal from UCAM

Umbilical cord was obtained. Blood was drained from the umbilical cord prior to freezing for storage.

The protocol from Example 7 was used - however, PBS 1X was substituted for 50% DMEM + 50% Glycerol.

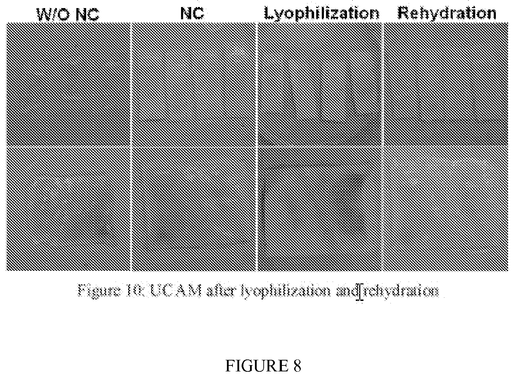

PBS 1X completely removed all traces of blood from the UCAM processed from an umbilical cord after 16 hours. *See* FIGURE 8. PBS 1X remove red blood cells at 1/8^{th} the time of 50% DMEM + 50% Glycerol and the reduced cost associated with using PBS 1X as compared to using 50% DMEM + 50% Glycerol.

### Example 12 - Optimization of Lyophilization

There were 5 experimental conditions tested for the lyophilization stage; i) w/o backing, ii) NM with stroma side down, iii) NM with stroma side up, iv) PTFE with stroma side down, and v) PTFE with stroma side up.

The UCAM was frozen with liquid nitrogen for 1 minute with a Petri dish as a barrier prior to lyophilization. Initial data has shown that the UCAM tends to freeze completely and attach strongly to the surface when it comes into physical contact with liquid nitrogen. In addition, excess liquid (i.e. PBS 1X) on the surface would also freeze with the UCAM making the whole UCAM/surface immobile. To solve this problem, a polyethylene Petri dish was placed in between the UCAM and liquid nitrogen to prevent any physical contact between the liquid and the tissue.

Next, the UCAM was lyophilized for 20 hours and subsequently rehydrated with PBS 1X. The weight of the UCAM during rehydration was recorded at 5, 10, 15, 30, 60 and 120 minutes interval.

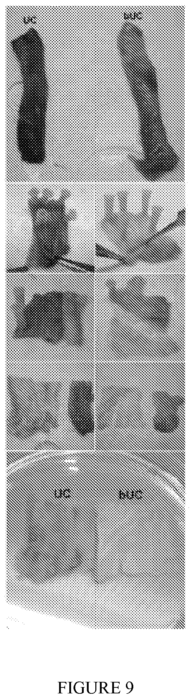

The UCAM adhered to NM with its sticky side up (epithelium side up) produces the flattest and nicest tissues after lyophilization (see FIGURE 9). The other UCAM with different experimental condition (w/o backing, on PTFE and on nylon membrane with stroma side down) were slightly shriveled up. It is also noted that all UCAM could be peeled off easily from its substrate after lyophilization.

We can also surmise that PTFE would not work as a backing for lyophilization as the PTFE membrane itself shrivels up during the process.

The lyophilized UCAM regained 54% and 71% (average) of its weight after 30 minutes and 60 minutes in PBS 1X respectively. The tissue also regained a lot of its structural integrity after 30 minutes as it could be easily stretched and manipulated.

| | **Without Backing** | **With Backing** | | | |
|---|---|---|---|---|---|
| | | **NM** | | **PTFE** | |
| | | **Stroma Down** | **Stroma Up** | **Stroma Down** | **Stroma Up** |
| **Initial Weight (g)** | .260 | 0.214 | 0.278 | 0.217 | 0.296 |
| **With Backing (g)** | ------- | 0.210 | 0.287 | 0.208 | 0.292 |
| **Liquid nitrogen (g)** | .246 | 0.198 | 0.297 | 0.210 | 0.288 |
| **Lyophilized (g)** | .015 | 0.035 | 0.030 | 0.033 | 0.028 |
| **5 Min AR (g)** | .111 | 0.118 | 0.097 | 0.109 | 0.109 |
| **10 Min AR (g)** | .138 | 0.142 | 0.134 | 0.134 | 0.151 |
| **15 Min AR (g)** | .147 | 0.154 | 0.135 | 0.132 | 0.168 |
| **30 Min AR (g)** | .131 | 0.180 | 0.155 | 0.148 | 0.172 |
| **1 Hour AR (g)** | .147 | 0.216 | 0.172 | 0.146 | 0.204 |

### Conclusions

Separation of UCAM and Wharton's Jelly is easier when sectioned Umbilical Cord is suspended in PBS 1X for a minimum of 30 minutes. PBS 1X is the buffer of choice to remove red blood cells from UCAM. UCAM should adhere to nylon membrane with its sticky side up prior to lyophilization. UCAM should be frozen prior to lyophilization with the aid of a Petri dish to prevent liquid nitrogen from contacting the tissue.

### Example 13 - Rehydration of Lyophilized UCAM

Lyophilized UCAM prepared according to Example 3 was used.

The lyophilized UCAM was placed in PBS 1X for 30 minutes.

### Example 14 - Protein Concentration in Isolated UCAM

Biological tissues containing UCAM were obtained from three donors. Extracts were made from the materials supplied by each donor: AM-UCAM; CH-chorionic membrane; AC-both AM and CH; UC-umbilical cord; PL-placenta.

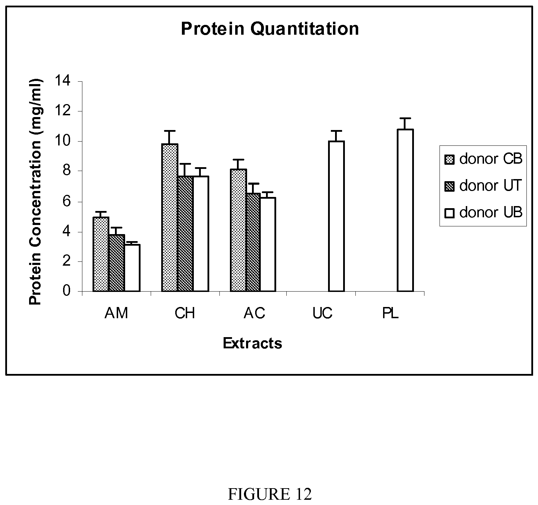

Protein concentrations in each extract were determined. (See also, FIGURE 12).

| **Extract** | **Protein Concentration (mg/ml)** | **Mean±SD (mg/ml)** | **P value** |
|---|---|---|---|
| **AM1** | 4.9 | 3.9±0.9 | - |
| **AM2** | 3.8 | | |
| **AM3** | 3.1 | | |
| **CH1** | 9.8 | 8.4±1.2 | 0.008502 (vs. AM) |
| **CH2** | 7.7 | | |
| **CH3** | 7.7 | | |
| **AC1** | 8.1 | 6.9±1.0 | 0.019539 (vs. AM) 0.186368 (vs. CH) |
| **AC2** | 6.5 | | |
| **AC3** | 6.2 | | |
| **UC1** | 10 | 10 | - |
| **PL1** | 10.8 | 10.8 | - |

Overall, the same tissue (AM, CH, and AC) generated similar protein concentrations from 3 donors, but AM generated a significant less protein concentration than that in CH, AC, UC, and PL.

### Example 15 - Suppression of cell viability (MTT Assay)

Biological tissues containing UCAM were obtained from three donors. Extracts were made from the materials supplied by each donor: AM-UCAM; CH-chorionic membrane; AC-both AM and CH; UC-umbilical cord; PL-placenta.

RAW264.7 mouse macrophage cells were harvested by a cell dissociation buffer. Cells were counted, resuspended, and seeded at 1x10⁵/ml (for simultaneous treatment by extracts) or 5x10⁴/ml (for sequential treatment by extracts). For simultaneous treatment, the cell suspension was mixed with 200 µg/ml proteins of each extract and cells were seeded for 3 h before stimulated with or without 1 µg/ml LPS for 24 h. For sequential treatment, cells were seeded for 24 first, followed by treatment with 200 µg/ml proteins of each extract for 3 h, then stimulated with or without 1 µg/ml LPS for 24 h. In both treatments, cells were subjected for cell viability (MTT) assay. During the period of treatment and incubation with MTT, cell morphology was also recorded by phase-contrast microscopy.

### A. Simultaneous treatment

### Morphology

There was no an apparent morphological difference between the control cells (PBS) and macrophage cells treated with 200 µg/ml proteins of extracts of UCAM (AM), chorion (CH), AM/AC [even umbilical cord (UC) and remaining placental tissues (PL)]. However, after MTT incubation for 4 h and before adding the solubilization buffer, the control cells were mostly round with the dark purple inside the cell body. In contrast, a majority of macrophage cells treated with extracts became needle-like cells. This phenomenon suggested that treatment of RAW264.7 mouse macrophage cells had changed the cell behavior (such as endocytosis and/or exocytosis) and led to a lower cell viability (or metabolic activity). The exact mechanism is not known. The control cells became more spread and larger after stimulation with LPS, but cells with different extract treatments inhibited LPS-induced cell spreading and enlargement.

### Cell viability

As shown in Figure 13A, compared to the control and without LPS stimulation, the cell viability was significantly suppressed by AM extracts from 3 donors (p values were 0.016, 0.006, and 0.005). The suppression was also significant by CH extracts (p<0.01), and was significantly more than AM extracts (p<0.01). AC extract also had a significant inhibitory effect (p < 0.01), which was significantly more than AM extracts but not CH extracts. With LPS stimulation, the suppression of cell viability had a similar pattern (Figure 13B).

### B. Sequential treatment (Fig. 13C and 13D)

### Morphology

Similar to the simultaneous treatment

### Cell viability

Similar to the simultaneous treatment

### C. Summary

Cell morphology was changed after treatment with 200 µg/ml proteins of extracts from AM, CH, and AC, particularly after MTT incubation and LPS stimulation.

All extracts of placental tissues (AM, CH, AC, UC, and PL) showed significant suppression of the cell viability. CH extract exhibited the highest potency in such suppression among all extracts based on the equivalent amount of proteins (200 µg/ml).

### Example 16 - Characterization of Components of UCAM Extracts (Western Blot)

Biological tissues containing UCAM were obtained from three donors. Extracts were made from the materials supplied by each donor: AM-UCAM; CH-chorionic membrane; AC-both AM and CH; UC-umbilical cord; PL-placenta.

Western blot results (Fig. 14) show that HC1, HC3 HC4, |α| and PTX3 were present in AM. lal and PTX3 are also present in CH, PL, and UC.

Note that UC has more PTX3 and interestingly CH does not have as much as PTX3 AM.

### Example 17- Use of a Pulverized UCAM product in the Treatment of Arthritis

An individual with arthritis in the joints of his hand is identified. A composition comprising pulverized UCAM and collagen is prepared and injected into the arthritic joints of the individual.

### Example 18- Use of a Pulverized UCAM product as a Dermal Filler

An individual with wrinkles around the eyes (i.e., Crow's feet) is identified. A composition comprising pulverized UCAM and hyaluronan is prepared and injected into the subdermal facial tissues surrounding the eyes.

### Example 19- Use of a UCAM product during Laminectomy

An individual in need of a laminectomy is identified. A tissue graft made of UCAM is prepared.

Laminectomy is performed. Following laminectomy, the tissue graft made of UCAM is place over the remaining vertebrae and affixed. The surgical site is closed and sutured.

### Example 20- Use of a UCAM product during Spinal Fusion

An individual in need of a spinal fusion is identified.

A composition comprising pulverized UCAM and morcelized bone tissue is prepared. The vertebrae to be fused are exposed. The composition_is injected between adjacent vertebrae. The surgical site is closed and sutured.

### Example 21- Use of a UCAM product to Repair Nerve Tissue

An individual in need of nerve repair is identified. A tissue graft made of UCAM is prepared.

The nerve to be repaired is exposed. The tissue graft made of UCAM is placed over damage to nerve and sutured in place. The surgical site is closed and sutured.

### Example 22- Use of a UCAM product to Repair a Hernia

An individual in need of spinal disc herniation repair is identified. A tissue graft made of UCAM is prepared.

The herniated spinal disc is exposed. In some embodiments, a UCAM product or UCAM product disclosed herein is used to repair a spinal disc herniation. The tissue graft made of UCAM is placed over damage to the annulus fibrosis and sutured in place. The surgical site is closed and sutured.

### Example 23- Use of a UCAM product to Repair a Torn Rotator Cuff

An individual in need of rotator cuff repair is identified. A tissue graft made of UCAM is prepared.

The torn supraspinatus tendon is exposed. The tendon is sutured together. The tissue graft made of UCAM is placed over the suture site and sutured into place. The surgical site is closed and sutured.

### Example 23- Use of a UCAM product to Repair Gingival Recession

An individual in need of gingival replacement is identified. A tissue graft made of UCAM is prepared.

The tissue graft of UCAM is placed over the area of gingival recession. The graft is sutured into place. A protective covering is place over the graft.

### Example 24- Use of a UCAM product in Coronary Artery Bypass

An individual in need of coronary artery bypass is identified. A tubular tissue graft made of UCAM is prepared by culturing it with a plurality of fibroblasts and endothelial cells.

The individual is placed on bypass. The atherosclerotic section of the artery is exposed and removed. The tubular tissue graft made of UCAM is sutured to the open ends of the artery such that the artery is rejoined. The surgical site is closed and sutured.

### Example 25- Use of a UCAM product in the Treatment of Glaucoma

An individual in need of a Glaucoma Drainage Device is identified. A tissue graft made of UCAM is prepared.

The Glaucoma Drainage Device is implanted into an eye. The tissue graft of UCAM is placed over the GDD and sutured into place. A protective contact lens is placed over the tissue graft.

### Example 26- Use of a UCAM product in the Treatment of a Diabetic Foot Ulcer

An individual with a diabetic foot ulcer is identified. A tissue graft made of UCAM is prepared.

The foot ulcer is debrided. The tissue graft is placed over the ulcer. A protective bandage is placed over the tissue graft.

### Example 27- Use of a UCAM product in the of a Burn

An individual with a 3^{rd} degree burn is identified. A tissue graft made of UCAM is prepared.

The burn is debrided. The tissue graft is placed over the burn. A protective bandage is placed over the tissue graft.

### Example 28- Use of a UCAM product following Removal of a Bladder Tumor

An individual with a bladder tumor is identified. A tissue graft made of UCAM is prepared.

The bladder is exposed. The tumor removed from the bladder. The tissue graft is placed over excision site. The surgical site is closed and sutured.

### Example 29- Use of a UCAM product to Supplement the Tympanic Membrane

An individual with a tympanic membrane is identified. A tissue graft made of UCAM is prepared.

The tympanic membrane is visualized. The tissue graft is placed over tear in the tympanic membrane and sutured into place. A protective covering is placed over the tissue graft.

## Claims

1. An isolated umbilical cord amniotic membrane (UCAM) product, wherein the UCAM product is a substantially flat sheet or a tubular sheet that does not comprise a vein or artery, cells with metabolic activity, active HIV-1, active HIV-2, active HTLV-1, active hepatitis B, active hepatitis C, active West Nile Virus, active cytomegalovirus, active human transmissible spongiform encephalopathy, or active *treponema pallidum,* wherein the natural structural integrity of the isolated UCAM is substantially preserved for at least 15 days after initial procurement, **characterized in that** the UCAM is from umbilical cord (UC), wherein said UC is initially processed i) by freezing it, or ii) by removing substantially all of the blood before said UC is frozen.

2. A UCAM product as claimed in claim 1, wherein the UCAM product is substantially free of Wharton's Jelly.

3. A UCAM product as claimed in claim 1, wherein the umbilical cord product is cryopreserved, lyophilized, terminally sterilized, or a combination thereof.

4. A UCAM product as claimed in any of claims 1 to 3 for use in promoting wound healing, reducing inflammation, reducing scarring, reducing angiogenesis, and/or reducing adhesion.

5. A UCAM product as claimed in any of claims 1 to 3 for use in repairing or supplementing damaged tissue, wherein the tissue was damaged due to a burn, a surgical incision, an area of necrosis resulting from an infection, trauma, a toxin, or a laceration.

6. A UCAM product as claimed in any of claims 1 to 3 for use as a protective covering over an ulcer, for example a venous stasis (VS) ulcer, a foot ulcer or a corneal ulcer.

7. A UCAM product as claimed in any of claims 1 to 3 for use as an anti-adhesion barrier.

8. A UCAM product as claimed in any of claims 1 to 3 for use in preventing adhesion in joint or tendon repairs.

9. A UCAM product as claimed in any of claims 1 to 3 for use in:
(i) preventing epidural fibrosis and/or scar adhesions following spinal surgery (e.g., laminectomy); or
(ii) preventing the development of proliferative scarring following spinal surgery (e.g., laminectomy); or
(iii) reducing or preventing the development of a postlaminectomy membrane; or
(iv) preventing the development of extradural compression or dural tethering following spinal surgery (e.g. laminectomy); or
(v) reducing or preventing the development of tethered nerve roots following spinal surgery (e.g., laminectomy); or
(vi) reducing or preventing the development of arachnoiditis following spinal surgery (e.g., laminectomy); or
(vii) repairing spinal disc herniation or as a protective graft over an incision or tear in a spinal disc; or
(viii) providing structural support in a spinal disc or replacing or supplementing a spinal disc; or
(ix) reducing or preventing migration of fibroblasts to the dura mater and collagen deposition on the dura mater following spinal surgery.

10. A UCAM product as claimed in any of claims 1 to 3 for use in:
i) repairing gingiva or repairing gingival recession;
ii) reconstructing, augmenting, or reinforcing gingiva; or
iii) preventing adhesion of soft tissue to gingiva.

11. A UCAM product as claimed in any of claims 1 to 3 for use in repairing, reconstructing, replacing, or supplementing damaged, compromised, or missing soft tissue.

12. A UCAM product as claimed in any of claims 1 to 3 for use in use in repairing, reconstructing, replacing, or supplementing damaged, compromised, or missing nerve.

## Patentansprüche

1. Isoliertes Produkt aus Nabelschnur-Amnionmembran (UCAM), wobei das UCAM-Produkt eine im Wesentlichen flache Lage oder eine röhrenförmige Lage ist, die keine Vene oder Arterie umfasst, Zellen mit Stoffwechselaktivität, aktives HIV-1, aktives HIV-2, aktives Hepatitis B, aktives Hepatitis C, aktives West-Nil-Virus, aktives Cytomegalovirus, aktive Transmissible Spongiforme Enzephalopathie oder aktives *Treponema pallidum,* wobei die natürliche strukturelle Integrität der isolierten UCAM nach erster Beschaffung im Wesentlichen mindestens 15 Tage konserviert wird, **dadurch gekennzeichnet, dass** die UCAM von Nabelschnur (UC) stammt, wobei die UC anfänglich behandelt wird durch i) Einfrieren dieser oder ii) Entfernen im Wesentlichen des gesamten Bluts, bevor die UC eingefroren wird.

2. UCAM-Produkt nach Anspruch 1, wobei das UCAM-Produkt im Wesentlichen frei ist von Wharton-Sulze.

3. UCAM-Produkt nach Anspruch 1, wobei das das Nabelschnurprodukt kryokonserviert, lyophilisiert, terminal sterilisiert oder eine Kombination davon ist.

4. UCAM-Produkt nach einem der Ansprüche 1 bis 3 zur Verwendung in der Förderung der Wundheilung, der Linderung von Entzündungen, der Reduzierung von Narbenbildungen, der Reduzierung von Angiogenese und/oder der Reduzierung von Adhäsionen.

5. UCAM-Produkt nach einem der Ansprüche 1 bis 3 zur Verwendung in der Rekonstruktion oder Ergänzung von verletztem Gewebe, wobei das Gewebe durch eine Verbrennung, einen chirurgischen Einschnitt, einen Nekrosebereich als Folge einer Entzündung, eines Traumas, eines Toxins oder einer Fleischwunde verletzt worden ist.

6. UCAM-Produkt nach einem der Ansprüche 1 bis 3 zur Verwendung als Schutzüberzug über einem Ulcus, wie etwa einem Ulcus cruris venosum, einem Fußulcus oder einem Hornhautgeschwür.

7. UCAM-Produkt nach einem der Ansprüche 1 bis 3 zur Verwendung als Antihaftsperre.

8. UCAM-Produkt nach einem der Ansprüche 1 bis 3 zur Verwendung in der Prävention von Adhäsion bei Rekonstruktionen von Gelenken oder Sehnen.

9. UCAM-Produkt nach einem der Ansprüche 1 bis 3 zur Verwendung in der:
(i) Prävention von epiduraler Fibrose und/oder Narbenverklebungen nach einer Wirbelsäulenchirurgie (z.B. Laminektomie); oder
(ii) Prävention der Entwicklung proliferativer Narbenbildung nach einer Wirbelsäulenchirurgie (z.B. Laminektomie); oder
(iii) Reduzierung oder Prävention der Entwicklung einer Postlamimektomie-Membran; oder
(iv) Prävention der Entwicklung extraduraler Kompression oder von duralem Tethering nach einer Wirbelsäulenchirurgie (z.B. Laminektomie); oder
(v) Reduzierung oder Prävention der Entwicklung anhaftender Nervenwurzeln nach einer Wirbelsäulenchirurgie (z.B. Laminektomie); oder
(vi) Reduzierung oder Prävention von Arachnoiditis nach einer Wirbelsäulenchirurgie (z.B. Laminektomie); oder
(vii) Rekonstruktion eines Bandscheibenvorfalls oder als schützendes Graft über einer Inzision oder einem Riss in einer Bandscheibe; oder
(viii) Bereitstellung einer Stützfunktion in einer Bandscheibe oder dem Ersatz oder der Ergänzung einer Bandscheibe; oder
(ix) Reduzierung oder Prävention von Fibroblasten zu der Dura Mater und der Kollagenablagerung an der Dura Mater nach einer Wirbelsäulenchirurgie.

10. UCAM-Produkt nach einem der Ansprüche 1 bis 3 zur Verwendung in der:
i) Rekonstruktion von Zahnfleisch oder der Rekonstruktion von Gingivarezession;
ii) Rekonstruktion, der Stärkung oder Verstärkung von Zahnfleisch; oder
iii) Prävention der Adhäsion von Weichgewebe an Zahnfleisch.

11. UCAM-Produkt nach einem der Ansprüche 1 bis 3 zur Verwendung in der Rekonstruktion, dem Ersatz oder der Ergänzung von verletztem, kompromittiertem oder fehlendem Weichgewebe.

12. UCAM-Produkt nach einem der Ansprüche 1 bis 3 zur Verwendung in der Rekonstruktion, dem Ersatz oder der Ergänzung von verletzten, kompromittierten oder fehlenden Nerven.

## Revendications

1. Produit de membrane amniotique de cordon ombilical (UCAM) isolée, le produit UCAM étant une feuille sensiblement plate ou une feuille tubulaire qui ne comprend pas de veine ou d'artère, de cellules ayant une activité métabolique, le VIH-1 actif, le VIH-2 actif, le HTLV-1 actif, l'hépatite B active, l'hépatite C active, le virus du Nil occidental actif, un cytomégalovirus actif, une encéphalopathie spongiforme humaine transmissible active, ou *treponema pallidum* actif, l'intégrité structurelle naturelle de l'UCAM isolée étant sensiblement préservée pendant au moins 15 jours après l'obtention initiale, **caractérisé en ce que** l'UCAM provient du cordon ombilical (UC), ledit UC étant initialement traité i) par congélation, ou ii) par élimination pratiquement totale du sang avant que ledit UC soit congelé.

2. Produit UCAM selon la revendication 1, le produit UCAM étant sensiblement exempt de gelée de Wharton.

3. Produit UCAM selon la revendication 1, le produit de cordon ombilical étant cryopréservé, lyophilisé, stérilisé en phase terminale, ou une combinaison de ceux-ci.

4. Produit UCAM selon l'une quelconque des revendications 1 à 3 destinée à être utilisé pour promouvoir la cicatrisation, réduire l'inflammation, réduire les cicatrices, réduire l'angiogenèse et/ou réduire l'adhérence.

5. Produit UCAM selon l'une quelconque des revendications 1 à 3, destiné à être utilisé pour réparer ou compléter un tissu endommagé, le tissu ayant été endommagé en raison d'une brûlure, d'une incision chirurgicale, d'une zone de nécrose résultant d'une infection, d'un trauma, d'une toxine, ou d'une lacération.

6. Produit UCAM selon l'une quelconque des revendications 1 à 3 destiné à être utilisé en tant que couverture protectrice sur un ulcère, par exemple un ulcère de stase veineuse (VS), un ulcère du pied ou un ulcère cornéen.

7. Produit UCAL selon l'une quelconque des revendications 1 à 3 destiné à être utilisé comme une barrière anti-adhérence.

8. Produit UCAM selon l'une quelconque des revendications 1 à 3 destiné à être utilisé dans la prévention de l'adhérence dans les réparations des articulations ou des tendons.

9. Produit UCAM selon l'une quelconque des revendications 1 à 3 destiné à être utilisé pour :
(i) prévenir la fibrose épidurale et/ou les adhérences cicatricielles après une chirurgie de la colonne vertébrale (p. ex. laminectomie) ; ou
(ii) prévenir le développement de cicatrices prolifératives après une chirurgie de la colonne vertébrale (p. ex. laminectomie) ; ou
(iii) réduire ou prévenir le développement d'une membrane post-laminectomie ; ou
(iv) prévenir le développement d'une compression extradurale ou d'un ancrage dural après une chirurgie de la colonne vertébrale (p. ex. laminectomie) ; ou
(v) réduire ou prévenir le développement de racines nerveuses ancrées après une chirurgie de la colonne vertébrale (p. ex. laminectomie) ; ou
(vi) réduire ou prévenir le développement de l'arachnoïdite après une chirurgie de la colonne vertébrale (p. ex. laminectomie) ; ou
(vii) réparer une hernie discale ou comme une greffe protectrice sur une incision ou une déchirure d'un disque vertébral ; ou
(viii) fournir un support structurel dans un disque vertébral ou remplacer ou compléter un disque vertébral ; ou
(ix) réduire ou prévenir la migration des fibroblastes vers la dure-mère et le dépôt de collagène sur la dure-mère après une chirurgie de la colonne vertébrale.

10. Produit UCAM selon l'une quelconque des revendications 1 à 3 destiné à être utilisé pour :
i) réparer la gencive ou réparer la récession gingivale ;
ii) reconstruire, augmenter ou renforcer la gencive ; ou
iii) prévenir l'adhérence de tissus mous à la gencive.

11. Produit UCAM selon l'une des revendications 1 à 3 destiné à être utilisé pour réparer, reconstruire, remplacer ou compléter des tissus mous endommagés, compromis ou manquants.

12. Produit UCAM selon l'une des revendications 1 à 3 destiné à être utilisé pour réparer, reconstruire, remplacer ou compléter un nerf endommagé, compromis ou manquant.
